# EUROPEAN PATENT APPLICATION

(11) **EP 2 777 712 A1**
(43) Date of publication of application: **17.09.2014**
(21) Application number: 12845273.7
(22) Date of filing: 02.11.2012
(51) Int. Cl.: A61K 39/095, A61K 39/112, A61P 37/04

(54) **ADJUVANTS FOR POLYSACCHARIDE VACCINES AND RESULTING FORMULATIONS**

(30) Priority: 02.11.2011 CU 20110202
(71) Applicant: Instituto Finlay. Centro de Investigación - Produccion de Sueros Y Vacunas, 11600 La Lisa, La Habana (CU)
(72) Inventor: PEREZ MARTÍN, Oliver Germán, Olaya, 11300 La Habana (CU); ROMEU ALVAREZ, Belkis, San Miguel del Padrón, 11000 La Habana (CU); LASTRE GONZÁLEZ, Miriam de San Juan Bosco, Playa, 11300 La Habana (CU); ZAYAS VIGNIER, Caridad, Marianao, 11500 La Habana (CU); GONZÁLEZ AZNAR, Elizabeth, La Lisa, 10600 La Habana (CU); BALBOA GONZÁLEZ, Julio Antonio, La Lisa, La Habana 10600 (CU); CABRERA BLANCO, Osmir, Playa, 11600 La Habana (CU); CUELLO PÉREZ, Maribel, Playa, 11600 La Habana (CU); GARCÍA IMIA, Luis Guillermo, Playa, La Habana (CU); ACEVEDO GROGUES, Reinaldo, Guanabacoa, 11100 La Habana (CU); BARBERÁ GONZÁLEZ, Ramón Faustino, Playa, 11300 La Habana (CU); PÉREZ QUIÑOY, José Luis, Playa, 11300 La Habana (CU); SOTOLONGO PADRÓN, Francklin Tomás, Playa, 11300 La Habana (CU); CAMPA HUERGO, Concepción, Playa, 11300 La Habana (CU); SIERRA GONZÁLEZ, Gustavo Victoriano, Playa, 11300 La Habana (CU); MANDIAROTE LLANES, Aleida, Playa, C., 11300 La Habana (CU); CARDOSO GONZÁLEZ, Daniel Tomás, Playa, 11300 La Habana (CU)
(74) Representative: Gil-Vega, Victor
(86) International application number: PCT/CU2012/000006
(87) International publication number: WO 2013/064129

(57) **Abstract**

The invention provides immunogenic compositions for mucosal and parenteral applications that consist of (1) single or multiple non conjugated bacterial polysaccharide and (2) potent adjuvants. Plain polysaccharides or olygosaccharides are preferred without excluding the addition of potent adjuvants to conjugated polysaccharides. It is preferred that adjuvants were AFCox (Adjuvant Finlay Cocleate family x) or AFPLx (Proteolyposome family x). Mucosal route is preferred without excluding the parenteral one or combinations of them. The invention also provides immunogenic compositions a) a capsular sacaride antigen from serogroup C or serogroup A from *Neisseria meningitidis,* b) adjuvants AFPL1 adsorbed unto aluminum by parenteral route or non adsorbed AFPL1 or AFCo1 by nasal route and c) the application of two simultaneous parenteral and mucosal 'priming' with any of the potent adjuvants formulated with the afore mentioned antigens. The use of potent adjuvants formulated with non conjugated polysaccharides enhance mucosal and systemic immune response, polarizing the independent thymus response of polysaccharides to a dependent thymus response with a pattern Th1, cellular, which ensures its functionability in young children and inducing immune memory without needing convalent conjugation.

## Description

All documents quoted hereinafter are added by reference.

### Related Patent requests

This patent is the continuation of the Patent request PCT/CU2003/000016 requested in Cuba on November 27, 2002 and published in English Language (WO 2004/047805 A1); of the Patent request PCT/CU2003/00007 requested on May 08, 2002 and published in English Language (WO 03/094964 A1); of the Patent request PCT /CU2004/000017 applied on 30/12/2003 and published in English Language (WO 2005063287 A1) ; of the Patent request PCT /CU2009/000008 requested on November 13, 2009 and published EP2359850 (A1), of the Patent request PCT/CU2011/000002 appliedon 2011 and of the Patent request CU/P/2011/123 requested on 31/5/2011.The aforementioned applications are included here by reference.

### FIELD OF THE TECHNIQUE

This invention is related to vaccinology, particularly to polysaccharide vaccines and adjuvants.

Its **technical objective** is to obtain immunogenic compositions for mucosal and parenteral applications comprising a single or multiple capsular bacterial saccharide preferably non conjugated able to induce immune memory in young children in order to use them in the pharmaceutical industry as vaccines in the treatment and prevention of human and veterinary diseases.

### STATE OF THE ART

According to the response induced, antigens are classified as Thymus dependent (TD), represented by proteins and T independent (TI). They are subdivided in TI-1 (Lypopolysaccharides, LPS or lipoolygosaccharides) and in TI-2 (Polysaccharides, Ps). Bacteria can be covered by a Ps capsule to protect from the imune response. The majority of Ps of pathogen bacteria ar negative charged or are neutral acting as antigens TI-2. A small number of them has positive and negative charges in their repetitive units ('zwitterionics') such as *Staphylococcus aureus* and *Streptococcus pneumonia* type 1 and they behave as TD antigens. The repetitive units of Ps TI directly interact with surface receptors (immunoglobulin) of lymphocytes B, without requiring or inducing response T. That is why, they do not work in newly born children whose marginal zones of the lymphatic nodules, where Ps are stored, are inmature. Consequently, the immune memory is not induced, which requires cellular response, T. Immune memory is the capacity the immune system has to remember a previous encounter with a pathogen or vaccine. Immune memory is the esential condition of vaccinology. It is only induced against TD antigens. However, there are several human plain Ps vaccines as the injectable tetravalent of serogroups A, C, Y and W135 (Armand et al. (1982) J. Biol. Stand.10:335-339. and Cadoz et al. (1985) Vaccine 3:340-342). Although they are effective in adolescents and adults, they induced a poor immune response, a short lasting protection in absence of memory and they can not be used in infants (ex. MMWR (1997) 46(RR-5) 1-10). Another problem of Ps is that they induced hyporesponse of antibodies in a booster dosse or in contact with the agent and they can even affect the memory induced against them. (ej. Granoff D and Pollard A J. The Pediatric Infectious Disease Journal 2007; 26(8):716-22).

The worldwide accepted strategy to solve TI of Ps is covalent conjugation. It forms stable and irreversible links between Ps and a carrier, generally a protein, that when providing epitopes T, turn conjugated TI antigens into TD, which ensures its function in newly born children , the induction of immune memory and the non induction of hyporesponse. The carrier activates cells T enhancing the expression of surface molecules important in the antigenic presentation and produces cytocines which are important to induce memory and to stimulate the production of antibodies TD. Conjugated olygosaccharides from *N. meningitidis* serogroup C have been proved for human use such as Menjugate® (Costantino et al. (1992) Vaccine 10:691-698). However, it is necessary to improve the conjugates of serogroups A, W135, Y and X as well as their manufacture. Nevertheless, Ps purification as well as its transformation in oligosaccharides is consolidated. (Frash (1990) p.123-145 of Advances in Biotechnological Processes vol. 13 (eds. Mizrahi & Van Wezel and WO 03/007985). Another unexplored and less accepted possibility, may be the presentation of Ps in an environment of potente adjuvants that solve TI by inducing immune memory and its funtioning in chuldren, particularly in those under 2 years old. A non covalent conjugation goes by weak links where the following forces intervene: Van der Waals, hydrophobic, hydrogen bridges and ionic interactions may be potentiated by potent adjuvants.

Adjuvants are substances that potentiate the specific immune response against an antigen, causing a faster induction and increasing its duration (Vogel FR. Dev. Biol. Stand. 1998,92:241-248).

Aluminum oxide is still the most used adjuvant in parenteral vaccines. However, it is not a potent adjuvant that might help new subunit vaccines. It i s considered that it only has property of delivery system and it has recently being described a new mechanism of independent action of Toll-like receptors involving Nalp3 (Eisenbarth SC et al. Nature, 2008,453:1122-1126). Neither its mucosal utility has been proved and it is assumed that it induces preferably a Th2 response. Licensed vaccinal adjuvants are few (MF59, ASO2, Virosoma, AFPL1, Proteollin, MPL, etc.) and fewer those with mucosal application, and none of them have been licensed in humans (LT mutants (LT63K, LTR72), CpG, Quitosana, ISCOM y AFCo1) (Singh M y O'Hagan DT. Pharm. Res. 2002, 19(6):715-728, Pérez O et al. Immunology and Cell Biology. 2004,82:603-610).

Adjuvants are involved in: stimulating the innate response (Immunopotentiators); distributing antigens properly (Delivery System) (Pashine A Valianti NM. Nat. Med. 2005,11:S63-68; Singh M y O'Hagan DT. Pharm. Res. 2002,19(6):715-728) and addressing the immune response to desired protective responses (Immunopolarization) (O Pérez, et al. Pharmacology Online, 2006,3:762-64). The development of immunopetentiators or delivery system that may be effective by mucosal route is another main objective aim of the vaccine development and of the world pharmaceutical companies. They has focused in searching a system of adjuvants that combines one or various immunipotentiators with an adequate delivery system. Otherwise, we have started from proteolyposomes and their cocleate derivatives that contain various immunopotentiators acting synergically, have delivery capacity and polarize the immune response preferably towars patterns Th1 with also induction of cytotoxic responses (CTL, Pérez O, et al. Scand J Immunology 2007,66:271-77; certificate of invention author OCPI 23313, 2008; Pérez Martin OG et al; WO/2004/047805; WO/2003/094964 and EP1716866). Cocleates developed by us are derived from proteolyposomes (outer membrane vesicles, mainly bacteria) containing proteins and lipids (proteolypidic structures) and that were addressed to obtain potent mucosal adjuvants. On the other hand, we have also developed proteolyposomes- non derived cocleates (Pérez Martin OG et al., OCPI, CU/P/2011/123). Globally, cocleates have also been obtained from synthetic or purified lipids (Gould-Fogerite et al. U. S. Pat. No 5, 643, 574, July 1, 1997; Zarif, Methods Enzymol 2005,391:314-29; Miclea, Varma et al. Biochim Biophys Acta 2007, 1768(11):2890-8; and Syed, Woo et al. Int J Pharm 2008, 363(1-2): 118-25).

We have found that polysaccharide from *N. meningitidis* serogroup C (PsC) coadministered with AFCo1 induces mucosal and systemic response in marines (Scand J Infect Dis, 2011, 1-5 and Expert Vaccine Review 2011). It has also been reported that agonists of TLR ('Toll-like receptor') generate a humoral response of long duration against plain Ps (*Streptococcus pneumoniae*) in adult and infant mice; but only when they are administered two days after Ps (Taillardet M, et al. JID 2010, 202:470-479), that is to say, they are two formulations with scarce vaccinal posibilities. In addition, the response that they potentiate, is specific IgG3 above all, subclass induced mainly by plain Ps and not by conjugated ones.

### Revelations of the invention

The invention provides immunogenic compositions for mucosal and parenteral applications comprising 1) a non conjugated single or multiple capsular bacterial saccharide and 2) potent adjuvants. Preferably the composition includes 3) one or o more additional antigens and/or 4) one or more adjuvants.

The invention also provides an immunogenic composition for parenteral application comprising saccharides of at least PsC and AFPL1 adsorbed unto aluminum oxide. In addition, the use of AFPL serogroup A and/or W135 from *Neisseria meni*ngitidis and the homologous Ps (PsA) without been restricted to them.

The invention also provides an immunogenic composition for mucosal application comprising saccharides of at least PsC or PsVi and AFPl1 without been restricted to them.

It is preferred that capsular Ps in the composition of the invention were non conjugated without excluding the conjugated ones, which permits the obtainment of fast formulations particularly important during epidemics.

The microenvironment of cytokines created by potente non inert adjuvants permits the collaboration B-T, its functionality in newly born children and infants and the induction of immune memory essential against Ps.

**The current invention aims** at obtaining immunogenic compositions for mucosal and parenteral formulations comprising a single or multiple bacterial capsular saccharide, preferably non conjugated, and potent adjuvants able to induce immune memory and to function in young children to use them in the treatment and prevention of human and veterinary diseases.

"Proteoliposome" is understood as the vesicles obtained from outer membranes of microorganisms, particularly bacteria using different methods, such as, isolation without detergent; a process including detergent (desoxycolate, SDS, etc.) or the extraction from vesicles ("bleb") from culture supernatants and particularly those revealed in US 5.597.572. Proteoliposomes consist of abundant proteins inserted in the lipidic structure.

"Adjuvantes Finlay" is understood as the families of AFPLx and AFCox.

"AFPLx" is understood as the family of Adjuvantes Finlay extracted from the outer membrane of microorganisms like AFPL1 from *N. meningitidis* serogroup B, AFPL2 from *Vibrio cholerae* and those obtained from *N. meningitidis* serogroup A or W 135, *Bordetella pertusis, Escherichia coli,* among others.

"AFCox" is understood as the family of cocleates derived from the respective proteoliposomes such as AFCo1 from *N. meningitidis* serogroup B, AFPL2 de *V. cholerae* among others.

"Proteoliposome-non derived cocleates" is understood as those neither derived from a proteliposome as referred in patent (WO/2004/047805), Pérez O et al. Infect Immun. 2001, 69(7):4502-4508 and Pérez O et al. Immunology and cell Biology. 2004, 82:603-610) nor those manufactured from synthetic lipids (Gould-Fogerite et al. U. S. Pat. No 5, 643, 574, July 1, 1997), but those referred in the applied patent (Pérez Martin OG and cols., CU/P/2011/123).

"Potent non inert adjuvants" is understood as AFPLx and AFCox derived or not from proteoliposomes that contain one or various "MAMP", particularly those of synergy action (such as LPS, porines and bacterial DNA traces among others) without limiting to these adjuvants and that contain various proteins inducing pro-inflammatory cytokines that polarize the response towards a predominant Th1 pattern.

"MAMP" is understood as molecular structures kept in pathogen or not microbes (known as PAMP), able to stimulate the innate immune system and with it, induce a potential adaptative response and even be involved in the polarization of the immune response.

The use of adjuvants containing various of synergic action is desirable when potent adjuvants are required. The line of adjuvant systems of GSK is based on the combination of several compounds with adjuvant effect, while "Adjuvant Finlay" derived from proteoliposomes are a good example of what has been previously patented or is in patent process by our group (Pérez Martin OG et al., OCPI, CU/P/2011/123), the current invention finds out that AFCo1 and AFPL1 are able to turn TI of Ps into TD and even more towards a cellular pattern, Th1.

The mucosal and parenteral systems are organized differently, and even the mucosal one is more compartmentalized. There are no mucosal adjuvants licensed in human vaccines and it is more difficult to induce response by mucosal route than by the parenteral one (Bouvet, J P et al. I & 1. 1994, 62:3957-3961 and Nardelli-Haefliger, DR, et al. J. Virol. 1999, 73:9609-9613). Nasal route was found to be as efficient as the parenteral one to induce memory and to function in newly born children and infant when using Ps adjuvated with referred potent adjuvants.

It is also part of the invention the obtainment of different vaccinal formulations that use the capacity of Adjuvantes Finlay of fuctioning by both routes: mucosal and parenteral to induce memory and functioning in newly born and infants against Ps and be applied in a single time vaccination strategy as described in applied patent (Single time vaccines: CU/P/2008/215).

TI antigens such as PsC and PsA serogroup C and A from *N. meningitidis,* respectively, and Ps Vi from *Salmonella typhi* were efficiently adjuvated by Adjuvantes Finlay. The combination A+C was also efficient as well as the homologous adjuvation of PsA by AFPL of serogroups A and W135. Similarly, Ps, as well as their oligosacaridic fractions were efficiently adjuvated. Memory response was induced in them in a booster dose several months after immunization.

Adjuvated Ps vaccinal formulations of the current invention can be used in prophylactic human and veterinary vaccines.

The **novelty** of the current invention relies on the obtainment of single adjuvated formulations of TI antigens that do not require convalent conjugation to break TI of Ps.

It is particularly **novel** the fact that it not only changes TI of Ps, but also it induces immune memory responses similar to conjugated Ps and functions in newlyborn and infants, who require potent stimuli due to the inmature characteristics of their immune systems.

It is particularly **novel** that the formulations function when apply by mucosal and/or parenteral routes or simultenously.

It is particularly **novel** that by this way the group of combined Ps vaccines can be increased.

### The proposed solution has the following advantages:

- Convalent conjugation, which is more expensive, is not required;
- Less expensive formulations of Ps and potent adjuvants are obtained which induce memory and function in newlyborn and infants, critical aspects of Ps;
- The elimination and final control of chemical products used in convalent conjugation is not required;
- Ps and adjuvants previously manufactured for their immediate formulations can be used during epidemics;
- It can be used in combined prophilactic and therapeutic vaccinal formulations by using various Ps or their oligosaccharides at the same time; and
- Apart from the systemic response induced by parenteral vaccines, specific IgAS (secretory) response, main mucosal protector, is added when applied by mucosal route or simultaneously muccosal and parenteral.

**The current invention will be described** by the following specific examples where the responses by parenteral (subcutaneous and intramuscular) and mucosal (intranasal) are compared using three polysaccharides: PsVi, PsC and PsA.

### Example 1. Obtainment of AFPL1 and AFCo1 formulations with polysaccharides from Nesisseria meningitidis serogroup C or polysaccharide Vi from Salmonella typhi

### AFColFormulation by close cycle

### Reagents and Solutions

- Resuspension Solution (RS): Tris 30 mM and 1% sodium desoxycolate, pH 7.6
- Formation Solution (FS): Tris 30 mM, CaCl₂10 mM and NaCl 100 mM, pH7.4
- Washing Solution (WS): Tris 10 mM, pH 7.2
- Proteoliposome from *N. meningitidis* serogroup B, lot 9012B manufactured by the Production Plant from Finlay Institute
- Polysaccharide C from *N. meningitidis* serogroup C and Polysaccharide Vi from *S*. *t*yphi, lots 31-IMC-9003 and 31-ESVi-001, respectively manufactured by Production Plant from Finlay Institute

### Metodology

- Proteoliposome from *N. meningitdis* is resuspended in the RS and it is adjusted at a concentration of 2 mg in 1 mL (10 mL)
- Incubate at 37 °C for 15 minutes
- Sonicate for 5 minutes
- Filter by 0.2 µm membranes
- For the formation process, the FS is added at the same of the resuspended proteoliposome (10 mL) at a flow of 8.3 mL per minutes in slow shaking
- Allow to stand for 15 to 30 minutes
- Add WS three times the reached volume after the formation process (60 mL)
- Centrifuge at 2200 rpm for 10 minutes
- Decant excess volume and leave final volume at the same formation volume reached, being the concentration of the formulation 1 mg en 1mL
- Observe by optical microscope Opton Standard 25
- Then polysaccharides are added by coadministration at a final concentration of 0.4 mg in 1 mL of PsC or 1 mg in 1 mL of PsVi.

### Results and conclusions

The formation of uniform small tubular structures in the case of AFCo1 is corroborated.

### Example 2. Inmunogenicity of intranasal formulation of AFCo1 and AFPL1 coadministered with polysaccharide Vi (PsVi) from Salmonella typhi

**Immunization protocol.** To evaluate immunogenicity of formulations, Balb/c mice were immunized with three intranasal (i.n) doses of 25 µL of AFPL1 or AFCo1 (50 µg) applied at 0, 7 and 14 days coadministered with 25 µg of PsVi. PsVi by i.n route at the same concentration and dose of formulations with AFPL1 and AFCo1, 25 µg of PsVi in 25 µL and the Cuban plain PsVi vaccine, Vax-TyVi^{®}, a 5 µg dose of PsVi in 100 µL i.m were used as control groups. A booster dose by i.m route was applied to all groups at 100 days of the last dose with the Cuban vaccine, Vax-TyVi (5 µg of PsVi in 100 µL).

**Obtainment method and sample processing.** Blood was extracted 15 days after the last dose. For serum obtainment , blood was extracted by puncture the retro-orbital plexus using heparinized capillaries. Obtained blood samples were incubated for 1 h at 37°C, then they were centrifuged at 12500 rpm for 10 min to extract the serum. It was then kept at -20°C until be used.

### IgG Detection by ELISA test

### Reagents and Solutions

- PsVi was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG and IgM conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology:

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of polysaccharide at a concentration de 10 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each of the diluted samples, incubate 2 h at 37°C;
- Wash three times with SL;
- Add 100 µL/well of Anti Mouse IgG and IgM conjugated to peroxidase diluted 1:2500 un SDS;
- Incubate 1 h at 37°C in wet chamber ;
- Wash five times with WS;
- Add 100 µL/well of a solution of hydrogen peorxide 0.01% (v/v) and 6 mg/mL of ortho-phenylendiamine (OPD) in SBS;
- Incubate in the dark for 30 min;
- The reaction is stopped by adding 50 µL of a Stopping solution and
- Absorbance measurement (OD) is carried out at a wavelenght of 492 nm in a microplate reader (Titertek, Multiskan Plus).

### Expression and calculation of results.

Results of anti PsVi IgG were expressed as OD. In this assay, samples are considered positive above the value of the means plus 2 standard deviations of the control samples (groups without immunization).

### Results

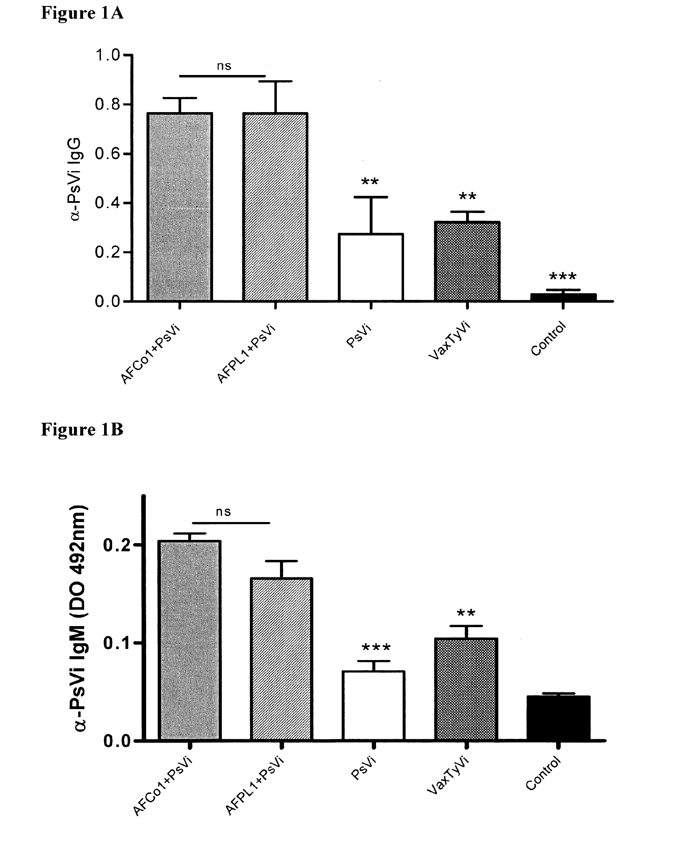

A systemic specific antigen response is induced, which is superior to the one reached by polysaccharide plain vaccine or by the polysaccharide administered by nasal route, with nasal formulations using adjuvants as stimulators of the anti-polysaccharide response, (Fig. 1A and 1B).

### Conclusions

Adjuvant AFPL1 and AFCo1 by nasa route are able to stimulate the specific antigen response against polysaccharide Vi.

### Example 3. Determination of the pattern of anti Polysaccharide IgG1, IgG3, IgG2a and IgG2c subclasses for intranasal formulation of AFPL1 or AFCo1 coadministered with PsVi

### Immunization protocol and sample collection . See example 2.

### Detection of anti Polysaccharide IgG1, IgG3, IgG2a and IgG2c subclasses by ELISA test

### Reagents and Solutions:

- PsVi was manufactured by the production plant from Finlay Institute, respectively, under the conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG3 and IgM conjugated to peroxidase ((Southern Biotech, Birmingham, EUA;
- biotinylated Anti Mouse -IgG1 and IgG2a (SIGMA, St. Louis, MO, EUA)
- Substrate buffer solution (SBS): Na₂HPO₄ 52 mM, citric acid 25 mM (pH 5,6) and
- Stopping solution: H₂SO₄ 2 M.

### Methodology:

- In 96- wells plates of high link capacity (Costar, EUA), add 100 µL/well of Poli-L-lisine at a concentration of 3 µg/mL, while PBSS is added and incubate 1 hour at room temperature in wet chamber ;
- In 96- wells plates of high link capacity (Costar, EUA), add 100 µL/well of polysaccharide at a concentration of 10 µg/mL while CBS is added and incubate overnight at 4°C in wet chamber ;
- Wash three times with PBSS ;
- Add 200 µL/well of BS and incubate 1 h at room temperature in wet chamber.
- Wash three times with WS;
- 1:100 in SDS and apply 100 µL/well in duplicate of each of the diluted samples, incubate 2 h at 37°C;
- Wash three times with WS;
- Add 100 µL/well of biotinylated anti mouse IgG1 and IgG2a conjugate diluted 1:5000 in SDS;
- Add 100 µL/well of anti mouse -IgG3 and IgG2c peroxidase conjugate diluted 1:6000 in SDS
- Incubate 2 h at 37°C in wet chamber ;
- Wash five times with WS;
- For IgG1 e IgG2a which are biotinylated, then wash plates three times and add 100 µL/well of the second conjugate (Sstreptavidine-peroxidase) 1:2500 in SDM and incubate 30 min at 37°C.
- Add 100 µL/well of a solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (OD) is carried out at a wavelenght of 492 nm in a microplate reader (Titertek, Multiskan Plus).

### Expression and calculation of results.

Results of anti PsVi IgG1, IgG3, IgG2a, IgG2c subclasses were expressed as OD. Results of anti PsVi IgG were expressed as OD. In this assay, samples are considered positive above the value of the means plus 3 standard deviations of the control samples (groups without immunization).

### Results

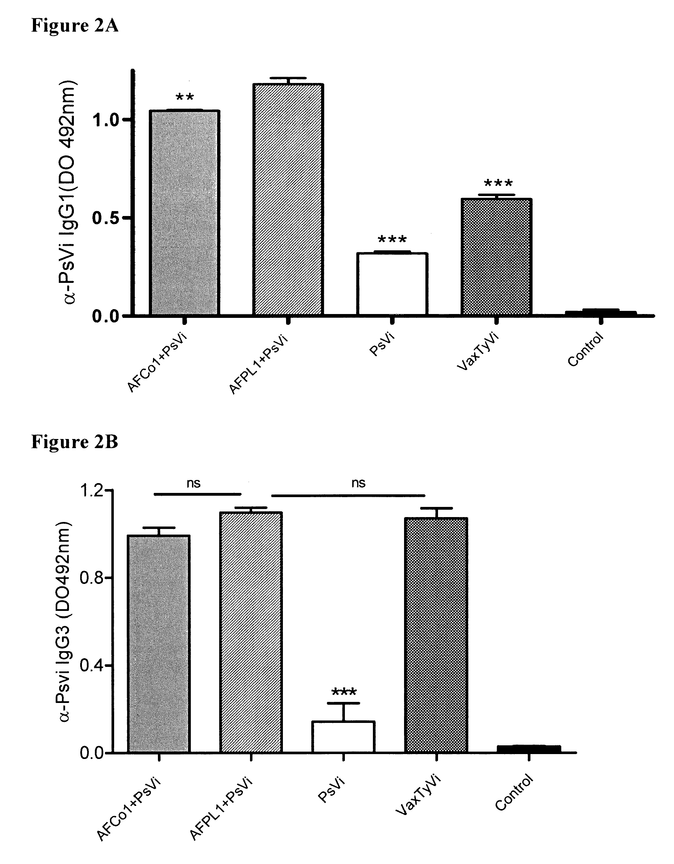

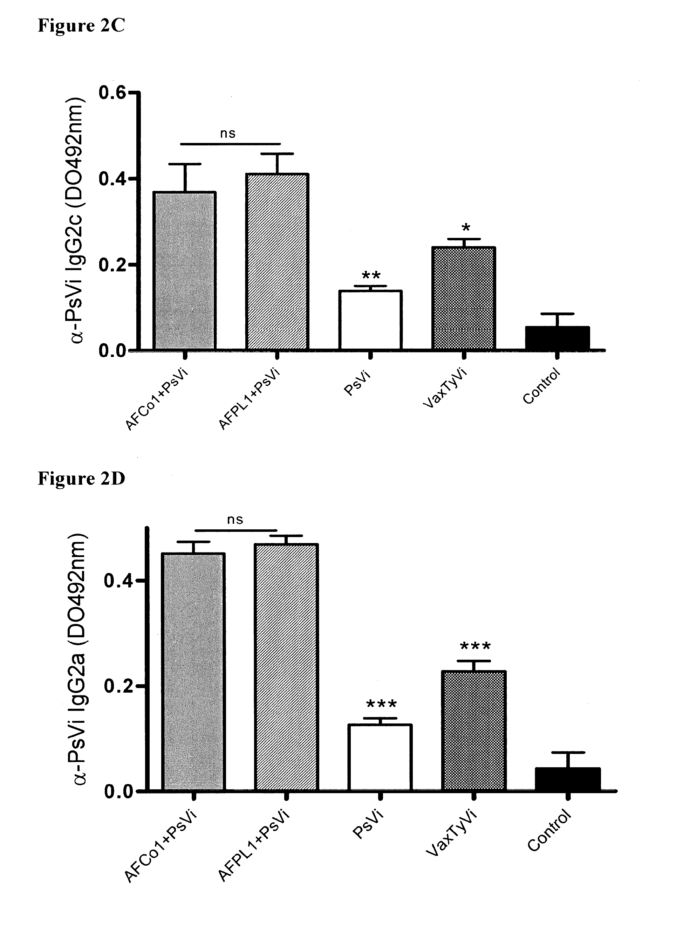

The use of AFPL1 and AFCo1 by nasal route induced high values of anti-PsVi IgG2c (Fig2C) and anti-PsVi Igg2a (Fig 2D) with the use of both adjuvants (Fig. 2A, 2B).

### Conclusions

Adjuvantes Finlay (AFPL1 and AFCo1) by nasal route permits the induction of a variate pattern of subclases, typical of TD antigen response. The induction of anti-PsVi IgG2a and anti PsVi IgG2c shows the induction of a cellular response, typical characteristic of TD antigens.

### Example 4. Determination of the affinity index and of the accumulated reduction percentage in different concentrations of Thiocyanate for intranasal formulation of AFPL1 or AFCo1 and PsVi

### Immunization Protocol and sample collection. See Example 2.

### Affinity detection of anti PsVi antibodies by avidity ELISA test

### Reagents and Solutions:

- PsVi was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Solution with chaotropic agent with Potassium Thiocyanate at different molar concentrations: KSCN 0.1M, 0.25M, 0.5M y 1M
- Anti Mouse IgG conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG and IgM conjugated to peroxidase (SIGMA, St. Louis, MO, EUA);
- Substrate buffer solution (SBS): Na₂HPO₄ 52 mM, citric acid 25 mM (pH 5,6) y
- Stopping solution: H₂SO₄ 2 M.

### Methodology:

- In 96- wells plates of high link capacity (Costar, EUA), add 100 µL/well of Poli-L-lisine at a concentration of 3 µg/mL, while PBSS is added and incubate 1 hour at room temperature in wet chamber ;
- In 96- wells plates of high link capacity (Costar, EUA), add 100 µL/well of polysaccharide at a concentration of 10 µg/mL for PsVi while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute sera 1:25 in SDS and apply 100 µL/well in duplicate of each of the diluted samples, incubate 2 h at 37°C;
- Wash three times with WS;
- Add 100 µL/well in duplicate of the different concentration of the chaotropic agent (0.1M, 0.25M, 0.5M and 1M) and a part of the plate is left with the samples that only have PBSS;
- Incubate at room temperature for 15 minutes;
- Wash three times with WS;
- Add 100 µL/well of anti mouse IgG peroxidase conjugate diluted 1:2500 in SDS
- Incubate 2 h at 37°C in wet chamber ;
- Wash five times with WS;
- Add 100 µL/well of a solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (OD) is carried out at a wavelenght of 492 nm in a microplate reader (Titertek, Multiskan Plus).

### Expression and calculation of results

The results of the affinity maturing of antibodies for a concentration of Potassium Thiocyanate were expressed as affinity index (%) that is calculated as the titer of the serum treated with Thiocyanate into the titer of serum not treated with Thiocyanate (PBS+Tween) by 100 and the reduction percentage of absorbance was expressed for each sample and with each Thiocyanate concentration

### Results

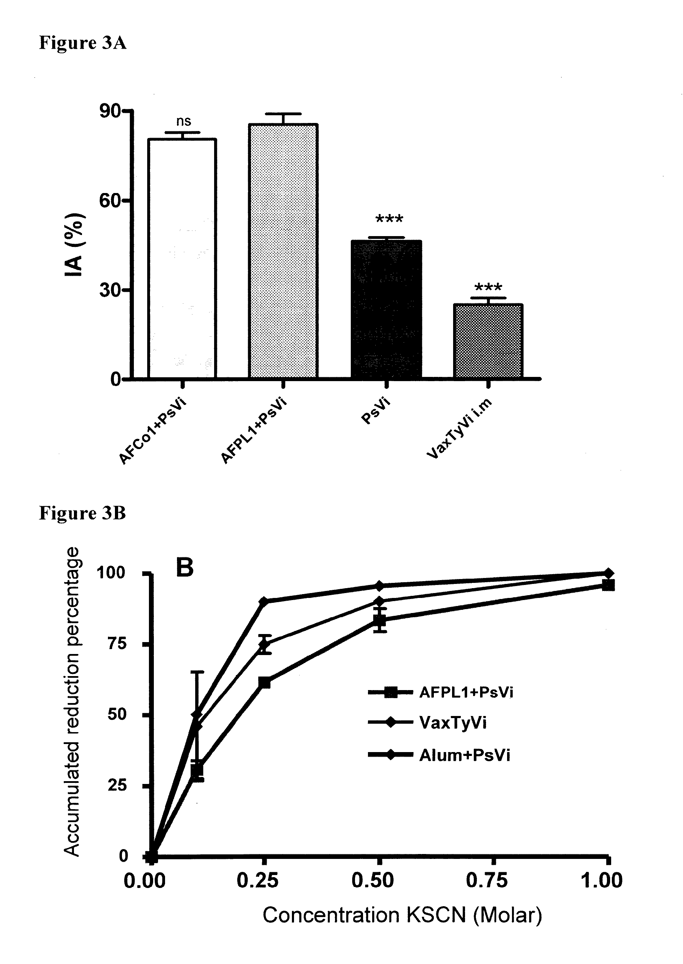

With both adjuvants and with polysaccharide by nasal route, an avidity index very superior to the one of polysaccharide vaccine or polysaccharide only by mucosal route is induced (Fig. 3A and 3B).

### Conclusion

The greater affinity of the serum samples of groups immunized i.n. with adjuvants Finlay and PsVi suggests a stronger formation of the antigen-antibody complex, maybe due to the costimulator signals of the adjuvant, which favor the formation of germinal centers leading to the proliferation and selction of a greater number of cells B of high affinity to the antigen epitopes, characteristic of TD antigens.

### Example 5. Kinetics of anti-polysaccharide antibodies of intranasal formulation dAFPL1 or AFCo1 coadministered with polysaccharide Vi (PsVi) from Salmonella typhi and secondary response after a booster dose with Vax-TyVi

### Immunization protocol. See Example 2.

**Obtainment method and sample processing.** 100 days after the last dose (Example 2), animals were challenged with an intramuscular dose of the Cuban polysaccharide Vi plain vaccine Vax-TyVi (5 µg/100 µL). For serum obtainment, blood was extracted by puncture of the retro-orbital plexus using heparinized capillaries. Blood was extracted at 15, 30, 60, 90, 100 and 112 days after the last dose. Obtained blood samples were incubated for 1 h at 37°C, then they were centrifuged at 12500 rpm for 10 min to extract the serum. Serum was stored at -20°C until use.

### anti PsVi IgG detection by ELISA test

### Reagents and Solutions

- PsVi was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Mouse Anti-IgG conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber ;
- In 96- wells plates of high link capacity (Costar, EUA), add 100 µL/well of polysaccharide at a concentration of 10 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each of the diluted samples, incubate 2 h at 37°C;
- Wash three times with WS;
- Add 100 µL/well of Mouse anti-IgG peroxidase conjugate diluted 1:2500 in SDS;
- Incubate 1 h at 37°C in wet chamber ;
- Wash five times with WS;
- Add 100 µL/well of a solution of hydrogen peroxide 0,01% (v/v) and 6 mg/mL of ortho-phenylenilendiamine (OPD) en SBS;
- Incubate in the dark for 30 min;
- The reaction is stopped by adding 50 µL of stopping solution and
- Absorbance measurement (OD) is carried out at a wavelenght of 492 nm in a microplate reader (Titertek, Multiskan Plus).

### Expression and calculation of results

Results of anti PsVi IgG of each of the measured times were expressed as OD. In this assay, samples are considered positive above the value of the means plus 2 standard deviations of the control samples (groups without immunization).

### Results

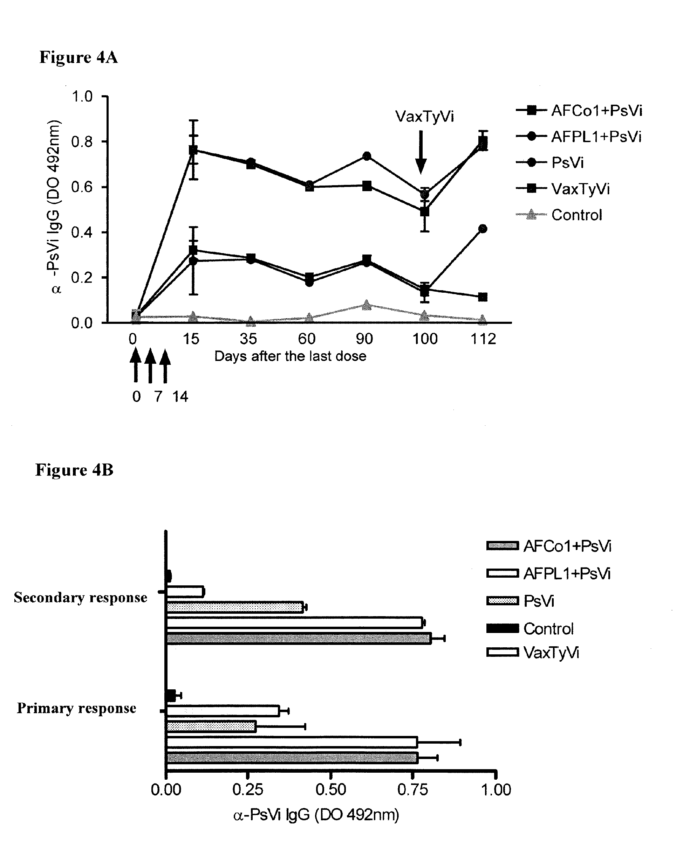

The secondary response of IgG antigen specific found out by an intramuscular dose with polysaccharide plain vaccine (Vax-TyVi) was superior in the formulations containing adjuvants by nasal route. (Fig. 4A and 4B).

### Conclusions

Antigens TI-2 are not able to reach a secondary response after repeated doses of plain polysaccharide. Primary immunization of PsVi with adjuvants AFPL1 and AFCo1 was able to develop a secondary immune response superior to the primary, distinctive feature of conjugated or protein vaccines, where repeated doses of antigen cause antibody titers similar or superior to those of the primary response. (booster response).

### Example 6. Determination of the affinity index and accumulated reduced affinity percentage for intranasal formulation of AFPL1 or AFCo1 and PsVi after a booster dose with VaxTyVi 100 days after the last dose

### Immunization Protocol and sample collection. See Example 2.

### Affinity detection if anti PsVi antibodies by avidity ELISA test

### Reagents and Solutions

- PsVi was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Solution with chaotropic agent with Potassium Thiocyanate at different molar concentrations: KSCN 0.1M, 0.25M, 0.5M and 1M
- Mouse Anti-IgG conjugated to peroxidase (SIGMA, St. Louis, MO, EUA);
- Substrate Buffer Solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine at a concentration of 3 µg/mL, while PBSS is added and incubate 1 hour at room temperature in wet chamber ;
- In 96- wells plates of high link capacity (Costar, EUA), add 100 µL/well of Polysaccharide at a concentration of 10 µg/mL for PsVi while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute sera 1:25 in SDS and apply 100 µL/well in duplicate of each of the diluted samples, incubate 2 h at 37°C;
- Wash three times with WS;
- Add 100 µL/well in duplicate the different concentrations of the chaotropic agent (0.1M, 0.25M, 0.5M and 1M) and a part of the plate is left with the samples that only have PBSS;
- Incubate at room temperature for 15 minutes;
- Wash three times with WS;
- Add 100 µL/well of the Mouse anti-IgG peroxidase conjugate diluted 1:2500 in SDS
- Incubate 2 h at 37°C in wet chamber ;
- Wash five times with WS;
- Add 100 µL/well of a solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (OD) is carried out at a wavelenght of 492 nm in a microplate reader (Titertek, Multiskan Plus).

### Expression and calculation of results

The results of the affinity maturing of antibodies for a concentration of Potassium Thiocyanate were expressed as affinity index (%) that is calculated as the titer of the serum treated with Thiocyanate into the titer of serum not treated with Thiocyanate (PBS+Tween) by 100 and the reduction percentage of absorbance was expressed for each sample and with each Thiocyanate concentration

### Results

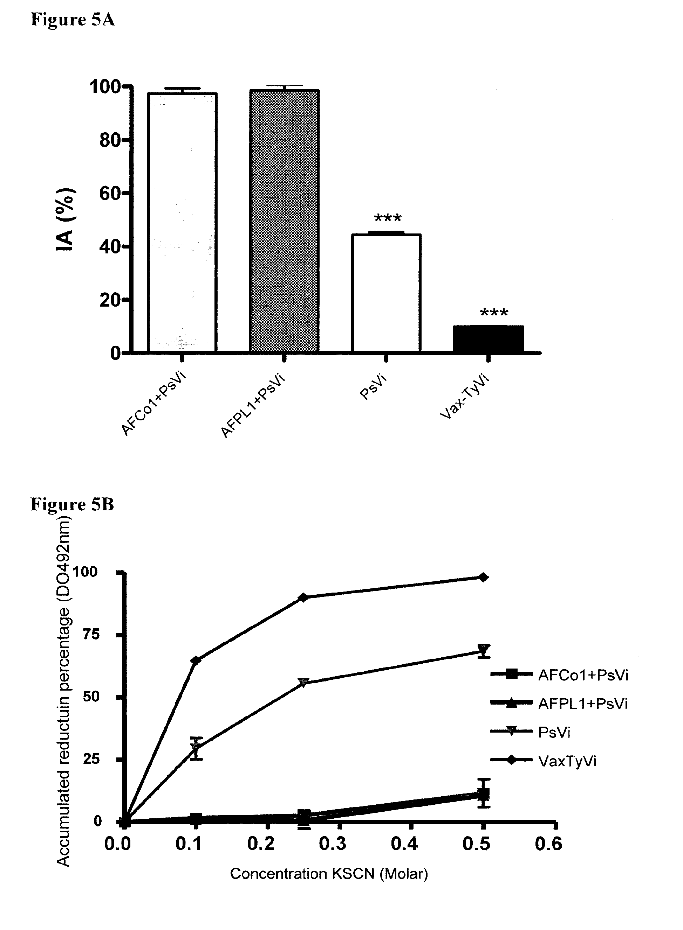

An affinity index superior to polysaccharide vaccine or immunization with polysaccharide only by mucosal was induced with both adjuvants and PsVi by nasal route. Obtained values of the affinity index are even superior to those obtained in the primary response (Fig. 5A y 5B).

### Conclusion

Antibodies produced by polysaccharide vaccines are less functional than antibodies induced by protein antigens. However, an increase in the affinity index after immunization with Vax-TyVi by parenteral route was observed in animals immunized with nasal adjuvants AFPL1 and AFCo1 and PsVi non conjugated convalently which suggests an induction of memory response of cells B. The immunization of a booster dose with the homologous antigen can cause that memory cells B of high affinity were recruited, even in the presence of antibodies these cells compite with virgin cells B of low affinity for the antigen, resulting in the production of high affinity antibodies.

### Example 7. Determination of the pattern of subclasses anti Polysaccharide IgG1, IgG3, IgG2a and IgG2c for intranasal formulation of AFPL1 or AFCo1 coadministered with PsVi after a booster dose with Vax-TyVi 100 days after the last dose.

### Immunization protocol, sample collection and booster dose. See Examples 2 and 5.

### Detection of subclasses anti PsVi IgG1, IgG3, IgG2a e IgG2c by ELISA test

### Reagents and Solutions

- PsVi was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG3 and IgG2c peroxidase conjugate (Southern Biotech, Birmingham, EUA);
- Anti Mouse biotinylated IgG1 and IgG2a (SIGMA, St. Louis, MO, EUA)
- Substrate Buffer Solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine at a concentration of 3 µg/mL, while PBSS is added and incubate 1 hour at room temperature in wet chamber ;
- In 96- wells plates of high link capacity (Costar, EUA), add 100 µL/well of polysaccharide at a concentration of 10 µg/mL while CBS is added and incubate overnight at 4°C in wet chamber ;
- Wash three times with PBSS;
- Anadir 200 µL/well de SB e Incubate 1 h at room temperature in wet chamber.
- Wash three times with WS;
- Dilute sera 1:100 in SDS and apply 100 µL/well in duplicate of each of the diluted samples, incubate 2 h at 37°C;
- Wash three times with WS;
- Add 100 µL/well of biotinylated anti Mouse anti-IgG1 and IgG2a conjugated diluted 1:5000 in SDS;
- Add 100 µL/well of anti Mouse IgG3 and IgG2c peroxidase diluted 1:6000 in SDS
- Incubate 2 h at 37°C in wet chamber;
- Wash five times with WS;
- For IgG1 and IgG2a, biotinylated, wash three times the plates and add 100 µL/well of the second conjugate (Streptavidine-peroxidase) 1:2500 in SDS and incubate for 30 min at 37°C.
- Add 100 µL/well of a solution of hydrogen peroxide 0,01% (v/v) and 6 mg/mL of ortho-phenylendiamine (OPD) in SBS;
- Incubate in the dark for 30 min;
- The reaction is stopped by adding 50 µL of a Stopping solution and
- Absorbance measurement (OD) is carried out at a wavelenght of 492 nm in a microplate reader (Titertek, Multiskan Plus).

### Expression and calculation of results.

The results of subclasses anti PsVi IgG1, IgG3, IgG2a, IgG2c were expressed as OD. In this assay, samples are considered positive above the value of the means plus 2 standard deviations of the control samples (groups without immunization).

### Results

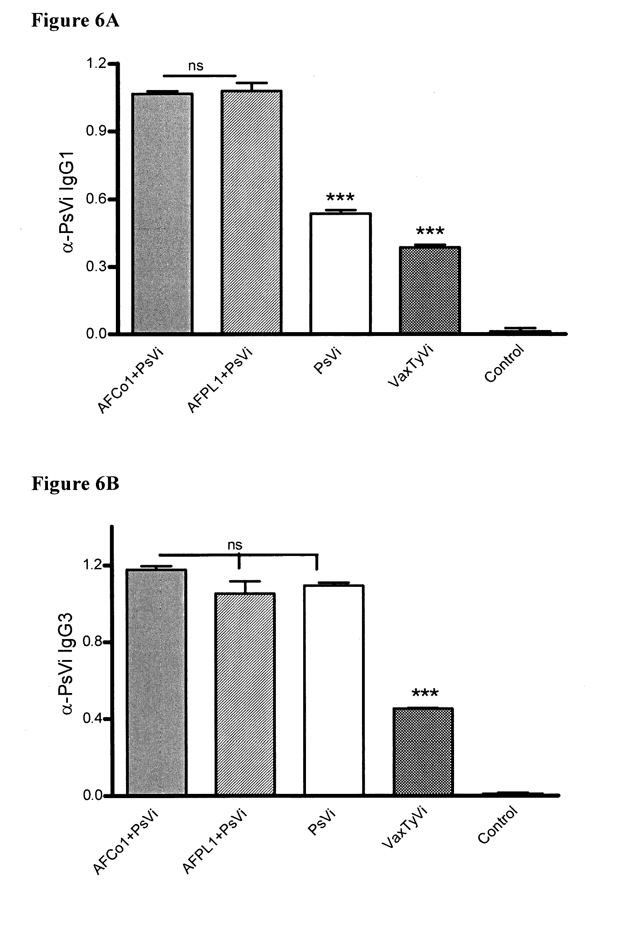

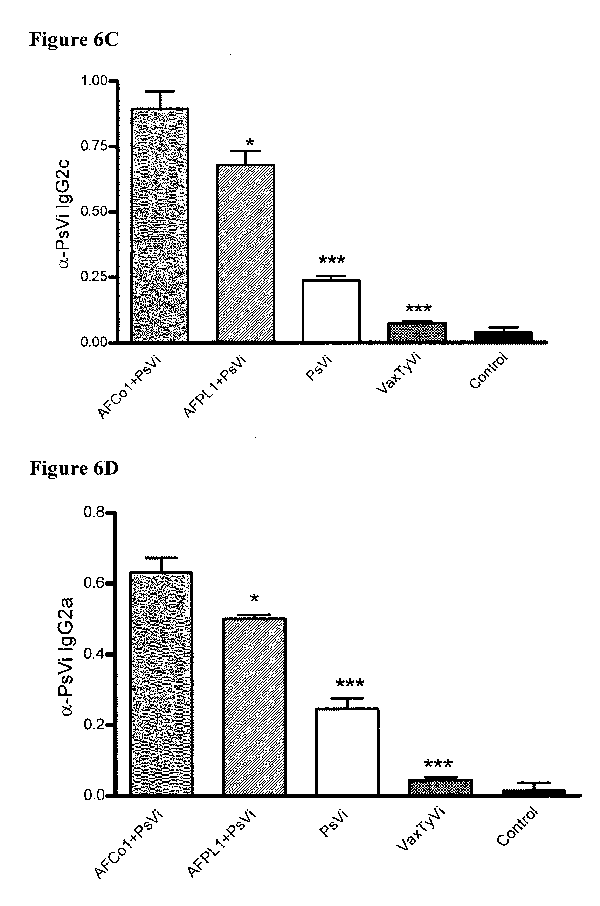

The response pattern induced with the use of both adjuvants in formulations by nasal route in the secondary response was similar to the pattern of subclasses anti-PsVi obtained in the primary response (Fig. 6A, 6B, 6C, 6D). The secondary response of anti-PsVi IgG2c and IgG2a was superior to primary response (Fig. 6C and 6D). The specific anti-PsVi IgG2a and IgG2c response was superior in the formulation containing AFCo1, as adjuvant. (Fig. 6C and 6D). The response against plain PsVi was characterized by high values of IgG3, typical of antigens TI-2 (Fig 6B).

### Conclusions

The subclass response of adjuvated formulations showed a pattern response f subclases superiot to the one obtained in primary response. Adjuvantes Finlay by nasal route allow the induction of non restricted pattern of subclases, typical of the response of antigens TD. The induction of values of anti polysaccharide IgG2a and IgG2c show the induction of a cellular response, typical characteristic of antigens TD.

### Example 8. Determination of effector T cells by standard ELISPOT after a booster dose with Vax-TyVi 100 days after the last dose.

### Immunization protocol, sample Collection and booster dose. See Examples 2 and 5.

### Reagents and Solutions

- Polysaccharide Vi was manufactured by the Production Plant from Finlay Institute, respectively, under conditions of good manufacturing practices;
- anti-mouse IFN-γ monoclonal antibody (BD Pharmingen, BD Bioscience)
- Culture medium DMEN (Sigma, St Louis, MO)
- Phosphate buffered saline solution (PBSS) 0.15 M (pH 7.2);
- Block solution (BS): Complete medium DMEN (fetal Bovine Serumo 10% and L-glutamine 0.29g/L) (SIGMA, St. Louis, MO, EUA);
- Washing solution 1 (SL1): PBSS;
- Washing Solution 2 (SL2): PBSS, 0,05% Tween-20 (v/v);
- biotinylated Anti-mouse IFN-γ (BD Pharmingen, BD Bioscience);
- Dilution solution (DS): SL, 1% BSA (SIGMA, St. Louis, MO, EUA);
- Solution of Acetate 0.1M (SA): Sodium acetate 0.2M and glacial acetic acid 0.2M , pH 5
- Solution of Aminoethylcarbazol (SAEC): 0.1mg of Aminoetilcarbazol (Sigma, St. Louis, MO, EUA) in 1 mL of Dimethyformamide (Sigma, St. Louis, MO, EUA)
- Substrate buffer solution (SBS): 800 µL of SAEC in 24 mL of SA, slow addition and moderate shaking, filter by 0.45 µm and add 12 µL of H₂O₂

### Methodology

- In 96- wells plates of high link capacity (Costar, EUA), add 100 µL/well of anti-mouse IFN-γ monoclonal antibody in PBSS (1/200) and incubate overnight at 4°C in wet chamber;
- Slaughter mice C57BL/6 and collect the spleens in complete DMEN medium (10% Bovine Fetal Serum and L-glutamine 0.29g/L)
- Wash three times with PBSS;
- Add 200 µL/well of BS and incubate 1 h at room temperature in wet chamber.
- Wash three times with WS 1;
- Add 1x10⁷ células of Spleen per well in triplicate in complete DMEN medium
- Stimulation of cells with 10µg/mL of PsVi, cells stimulated with Concavaline A are used as positive controls (ConA 5 µg/mL) while only with culture medium the negative controls.
- Incubate 24 hours at 37°C and 5% of CO₂
- Wash three times with WS 1;
- Wash three times with WS 2;
- Add 100 µL/well anti-mouse IFN-γ monoclonal antibody at a working concentration of 1/1000 in DS;
- Incubate overnight at 4°C in wet chamber ;
- Wash three times with WS 2;
- Wash three times with WS 1;
- Add 100 µL/well of the second conjugate (Streptavidine-peroxidase) 1:2500 in DS and incubate for 2 hours at 37°C.
- Wash three times with WS 2;
- Wash three times with WS 1;
- Add 100 µL/well of solution SBS;
- Incubate in the dark for 30 min;
- Determination and spot counting by stereomicroscope (Opton, Stemi V11).

### Expression and calculation of results

The results of anti-PsVi IFN-γ secretory cells were expressed as the number of cells that secret IFN-γ when they are stimulated with PsVix10⁵.

### Results

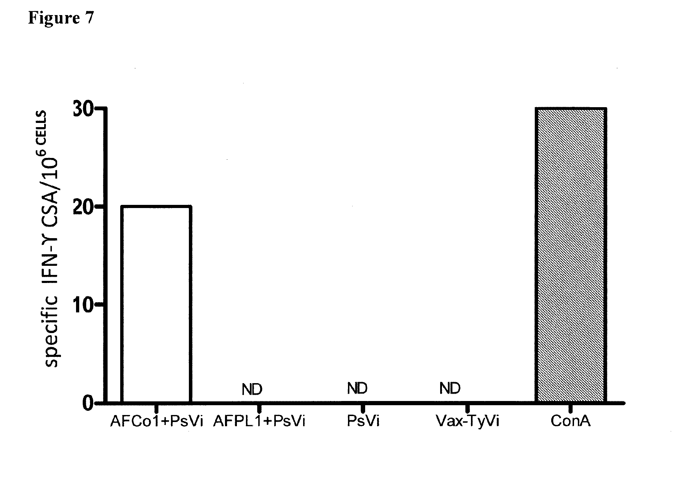

In the formulation with adjuvant AFCo1 a response of anti PsVi IFN-γ secretory cells was detected after a booster dose with the homologous without conjugation by parenteral route (Fig. 7).

### Conclusions

Antigens TI-2 like plain Ps are unable of stimulating a response of T cells, since they only activate B cells in absence of cooperation of T cells. Only conjugated Ps vaccines are able to stimulate the activation of T cells, due to the presence of the carrier protein in the conjugate. The detection of a anti PsVi IFN-γ response after a booster dose with the homologous antigen was extremely surprising because the primary immunization was carried out with formulations that did not included covalent conjugation, which confirms that the use of potent adjuvants in formulations with antigens TI-2 may revert thus thymus-dependence, acting like a conjugated vaccine.

### Example 9. Systemic immunogenicity of intranasal formulation of AFCo1 coadministered with polysaccharide from Neisseria meningitidis serogroup C (PsC) after a nasal booster with PsC

**Immunization protocol.** To evaluate immunogenicity of formulations after an intranasal booster dose with plain PsC 70 days after the last dose, adult Balb/c mice were immunized with three 25 µL intranasal (i.n) doses of AFCo1 (50 µg) coadministered with 10 µg of PsC at 0, 7 and 14 days. PsC only was used as control group at a final concentration and dose similar to the formulation with AFCo1. Both groups received a booster dose by i.n route 70 days after the last dose with plain PsC (10 µg de PsC in 25 µL).

**Obtainment method and sample processing.** Blood was extracted 21 days after the last dose and 21 days after the booster i.n only with PsC . To obtain the serum, blood was extracted by puncture retro-orbital plexus using heparinized capillaries. Obtained blood samples were incubated for 1 h at 37°C, then they were centrifuged at 12500 rpm for 10 min to extract the serum. Serum was stored at -20°C until use.

### Anti PsC IgG Detection by ELISA

### Reagents and Solutions

- Polysaccharide C was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber ;
- In 96- wells plates of high link capacity (Costar, EUA), add 100 µL/well of Polysaccharide at a concentration of 2.5 µg/mL for PsC while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each of the diluted samples, incubate 2 h at 37°C;
- Wash three times with PBSS;
- Add 100 µL/well of anti mouse IgG peroxidase conjugate diluted 1:2500 in SDS;
- Incubate 1 h at 37°C in wet chamber ;
- Wash five times with WS;
- Add 100 µL/well of a solution of hydrogen peroxide 0,01% (v/v) and 6 mg/mL of ortho-phenylendiamine (OPD) en SBS;
- Incubate in the dark for 30 min;
- The reaction is stopped by adding 50 µL of stopping solution and
- Absorbance measurement (OD) is carried out at a wavelenght of 492 nm in a microplate reader (Titertek, Multiskan Plus).

### Expression and calculation of results

Results of anti PsC IgG were expressed as OD. In this assay, samples are considered positive above the value of the means plus 2 standard deviations of the control samples (groups without immunization).

### Resultados

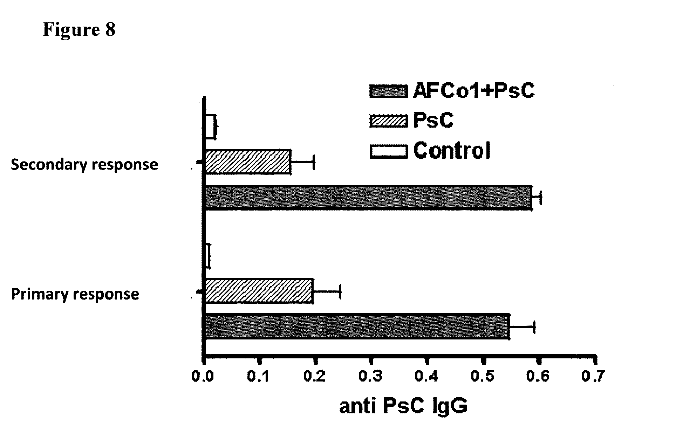

The anti PsC IgG secondary response observed after intranasal immunization of plain PsC adjuvated with AFCo1 was similar to the primary response in formulation containing the adjuvant by nasal route. (Fig. 8).

### Conclusions

Antigens TI-2 like plain Ps are unable of inducing a secondary memory response after repeated doses of plain polysaccharide. Primary immunization of PsC with adjuvant AFCo1 was able to develop a secondary immune response similar to the primary response, a distinctive characteristic of conjugated or protein vaccines, where repeated doses of the antigen cause antiboy titers similar or superior to primary response (booster response).

### Example 10. Mucosal Immunogenicity of intranasal of AFCo1 coadministered with polysaccharide C from Neisseria meningitidis (PsC) after a nasal booster dose with plain PsC.

### Immunization protocol. See example 9.

**Obtainment method and sample processing.** Saliva extraction and vaginal douching were carried out at 7 and 28 days after the last dose, respectively. Similarly was done 7 and 28 days after the booster dose i.n with only PsC. For the obtainment of saliva, mice were immunized with 100µL of pylocarpine 0.3% to stimulate salivation. Saliva was collected in pool, in cold and then proteases were inactivated in thermosthated bath at 56°C for 15 minutes. Vaginal douching of mice was carried out with 50 µL of PBSS three times. Saliva and vaginal samples were centrifuged at 10 000 rpm for 10 minutes and stored at -20°C until use.

### Anti PsC IgA Detection by ELISA in saliva and vaginal douching

### Reagents and solutions

- Ps C was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse biotinylated IgA (SIGMA, St. Louis, MO, EUA);
- Streptavidine peroxidase conjugate (SIGMA, St. Louis, MO, EUA);
- Substrate Buffer Solution (SBTS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber ;
- In 96- wells plates of high link capacity (Costar, EUA), add 100 µL/well of Polysaccharide at a concentration of 2.5 µg/mL for PsC while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute saliva and vaginal douching 1:10 in SDS and apply 100 µL/well in duplicate of each of the diluted samples, incubate 2 h at 37°C;
- Wash three times with WS;
- Add 100 µL/well of anti Mouse biotinylated IgA conjugate diluted in a concentration of 2µg/mL in SDS;
- Incubate 1 h at 37°C in wet chamber;
- Wash five times with WS;
- Since IgA is biotinylated, then wash the plates three times and add 100 µL/well of the second conjugate (Streptavidine-peroxidase) 1:2500 in SDS and incubate for 30 min at 37°C.
- Add 100 µL/well of solution of hydrogen peroxide 0,01% (v/v) and 6 mg/mL of ortho-phenylendiamine (OPD) en SBS;
- Incubate in the dark for 30 min;
- The reaction is stopped by adding 50 µL of a stopping solution and
- Absorbance measurement (DO) is carried out at a wavelength of 492 nm in a microplate reader (Titertek, Multiskan Plus).

### Expression and calculation of results

Results of anti PsC IgA were expressed as OD. In this assay, samples are considered positive above the mean values plus 3 standard deviations of control samples (groups without immunization).

### Results

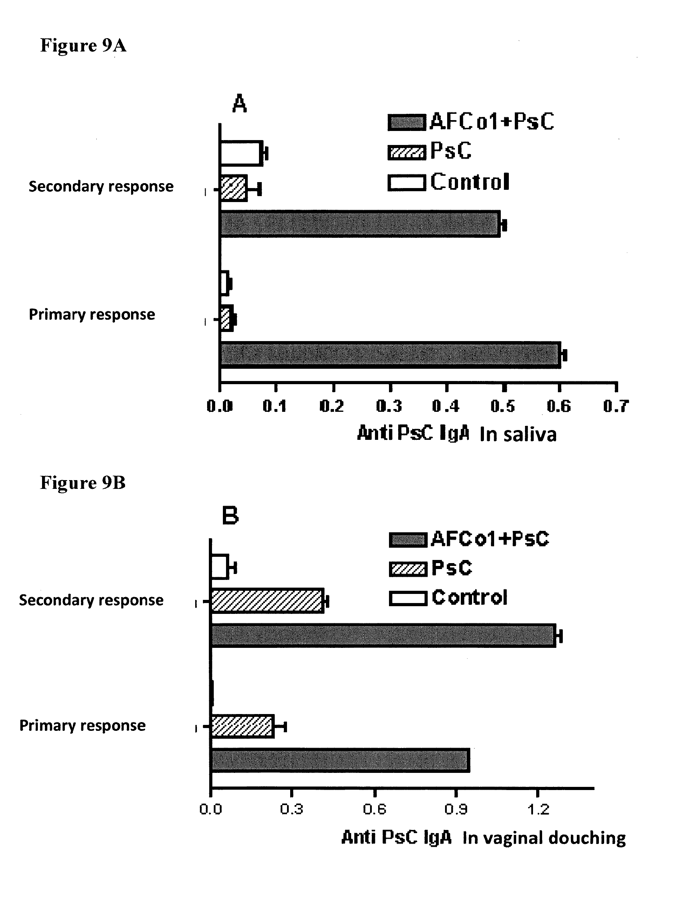

Specific antigen IgA secondary response was detected in saliva (Fig. 9A) and vaginal samples after intranasal immunization of plain PsC (Fig. 9B).

### Conclusions

TI-2 antigens are unable to reach a secondary response after repeated doses of plain polysaccharides. Primary immunization of PsC with adjuvant AFCo1 was able to develop a secondary immune response similar to the primary response, a distinctive characteristic of conjugated or protein vaccines, where antigen repeated doses cause antibody titers similar or superior to primary responses (booster response). The detection of IgA levels in both mucosal surfaces suggest that we are reactivating B cell clones involved in IgA response that were activated during the primary response at mucosal level, being this response superior when the adjuvant is present in the formulation. The detection of this antibody at mucosal level plays an important in the reduction of mucosal colonization.

### Example 11. Determination of the pattern of anti Polysaccharide IgG1, IgG2a subclasses intranasal formulation of AFCo1 coadministered with PsC after a nasal booster with plain PsC

### Immunization protocol. See example 9.

### Detection of anti Polysaccharide IgG1, IgG2a Subclasses by ELISA test

### Reagents and solutions

- Polysaccharide C was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse biotinylated IgG1 and IgG2a (SIGMA, St. Louis, MO, EUA)
- Substrate buffer solution (STS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber ;
- In 96- wells plates of high link capacity (Costar, EUA), add 100 µL/well of Polysaccharide at a concentration of 2.5 µg/mL for PsC while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute serum 1:100 in SDS and apply 100 µL/well in duplicate of each diluted samples and incubate 2 h at 37°C;
- Wash three times with WS;
- Add 100 µL/well of anti mouse biotinylated IgG1 and IgG2a diluted 1:5000 in SDS;
- Incubate 2 h at 37°C in wet chamber ;
- Wash five times with WS;
- For IgG1 and IgG2a that are biotinylated, then wash plates three times and add 100 µL/well of the second conjugate (Streptavidine-peroxidase) 1:2500 in SDS and incubate for 30 min at 37°C.
- Add 100 µL/well of solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (DO) is carried out at a wavelength of 492 nm in a microplate reader (Titertek, Multiskan Plus).

### Expression and Calculation of results

Results of anti PsC IgG1 and IgG2a subclasses were expressed as OD. In this assay, samples are considered positive above the mean values plus 2 standard deviations of control samples (groups without immunization).

### Results

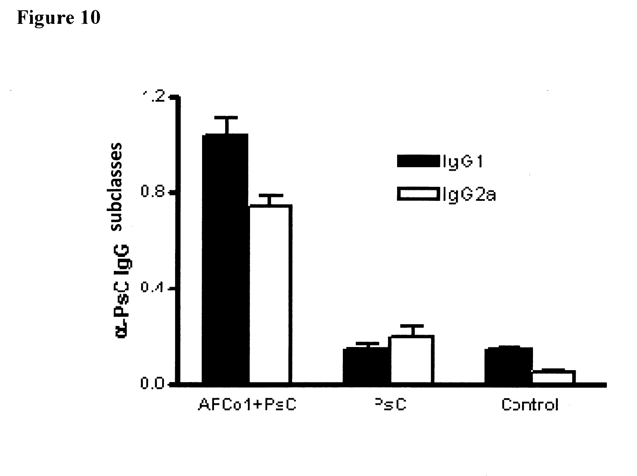

A secondary response of anti PsC IgG1 and IgG2a subclasses after intranasal immunization of plain PsC adjuvated with AFCo1 (Fig. 10).

### Conclusions

IgG2a response detected in animals immunized with PsC and AFCo1 suggests the induction of cellular response, characteristic mainly observed with TD antigens or conjugated Ps.

### Example 12. Immunogenicity of intramuscular formulation of AFPL1 coadministered with polysaccharide Vi from Salmonella typhi (PsVi)

**Immunization protocol.** To evaluate immunogenicity of the formulations, Balb/c mice were immunized with a 100 µL intramuscular (i.m) dose of AFPL1 (12 µg) coadministered with 5 µg of PsVi. PsVi adsorbed unto aluminum oxide at a final concentration like the formulation with and the Cuban PsVi plain vaccine, Vax-TyVi^{®} were used as control groups, at a 5 µg dose of PsVi in 100 µL i.m. All groups received a booster dose i.m 100 days after the last dose with the Cuban vaccine, Vax-TyVi (5 µg de PsVi in 100 µL).

**Obtainment method and sample processing.** Blood was extracted 15 days after the last dose. For serum obtainment, blood was extracted by puncture of the retro-orbital plexus using heparinized capillaries. Obtained blood samples were incubated for 1 h at 37°C, then they were centrifuged at 12500 rpm for 10 min to extract serum. Serum was stored at -20°C until use.

### Anti PsVi IgG and IgM Detection by ELISA test

### Reagents and solutions

- PsVi was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti mouse IgG conjugated to peroxidase (SIGMA, St. Louis, MO, EUA);
- Anti mouse IgM conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of Polysaccharide at a concentration of 10 µg/mL for PsVi while CBS is added and incubate overnight at 4°C in wet chamber ;
- Wash three times with PBSS;
- Add 200 µL/well of SB and incubate 1 h at room temperature in wet chamber.
- Wash three times with WS;
- Dilute sera 1:100 in SDS and apply 100 µL/well in duplicate of each diluted samples and incubate 2 h at 37°C;
- Wash three times with WS;
- Add 100 µL/well of anti mouse IgG peroxidase conjugate diluted 1:2500 or anti mouse IgM peroxidase conjugate diluted 1:2500 in SDS;
- Incubate 1 h at 37°C in wet chamber ;
- Wash five times with WS;
- Add 100 µL/well of solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (DO) is carried out at a wavelength of 492 nm in a microplate reader (Titertek, Multiskan Plus).

### Expression and Calculation of results

Results of anti PsVi IgG were expressed as OD. In this assay, samples are considered positive above the mean values plus 3 standard deviations of control samples (groups without immunization).

### Results

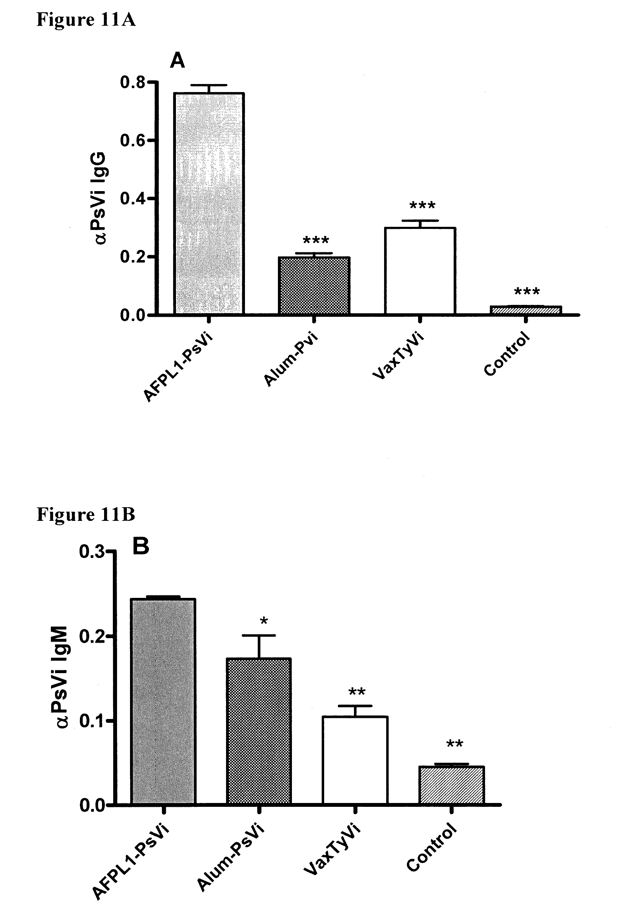

A specific antigen systemic response remarkably superior to the one reached by plain polysaccharide vaccine or the formulation of polysaccharide with aluminum hydroxide as adjuvant (Fig. 11A and 11B).

### Conclusions

The predominant immune response produced by polysaccharide vaccines is IgM type, where there is a very few specific IgG response. Adjuvant AFPL1 by intramuscular route is able to stimulate a high specific antigen IgG systemic response against PsVi.

### Example 13. Determination of pattern of anti Polysaccharide IgG1, IgG3, IgG2a and IgG2c subclasses for intramuscular formulation of AFPL1 and PsVi

### Immunization protocol and sample collection. See example 12.

### Detection of anti Polysaccharide IgG1, IgG3, IgG2a and IgG2c subclasses by ELISA test

### Reagents and solutions

- Polysaccharide Vi was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti mouse IgG3 and IgG2c conjugated to peroxidase (Southern Biotech, Birmingham, EUA);
- Anti mouse biotinylated IgG1 and IgG2a (SIGMA, St. Louis, MO, EUA)
- Substrate buffer solution (STS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of Polysaccharide at a concentration of 10 µg/mL while CBS is added and incubate overnight at 4°C in wet chamber ;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each diluted samples and incubate 2 h at 37°C;
- Wash three times with WS;
- Add 100 µL/well of anti mouse biotinylated IgG1 and IgG2a conjugated diluted 1:5000 in SDS;
- Add 100 µL/well of anti mouse IgG3 and IgG2c peroxidase conjugate diluted 1:6000 in SDS
- Incubate 2 h at 37°C in wet chamber;
- Wash five times with WS;
- For IgG1 and IgG2a that are biotinylated, then wash plates three and add 100 µL/well of the second conjugate (Streptavidine-peroxidase) 1:2500 in SDS and incubate forr 30 min at 37°C.
- Add 100 µL/well of solution of hydrogen peroxide 0,01% (v/v) and 6 mg/mL of ortho-phenylendiamine (OPD) in STS;
- Incubate in the dark for 30 min;
- The reaction is stopped by adding 50 µL of stopping solution and
- Absorbance measurement (DO) is carried out at a wavelength of 492 nm in a microplate reader (Titertek, Multiskan Plus).

### Expression and Calculation of results

Results of anti PsVi IgG1, IgG3, IgG2a, IgG2c subclasses were expressed as OD. In this assay, samples are considered positive above the mean values plus 3 standard deviations of control samples (groups without immunization).

### Results

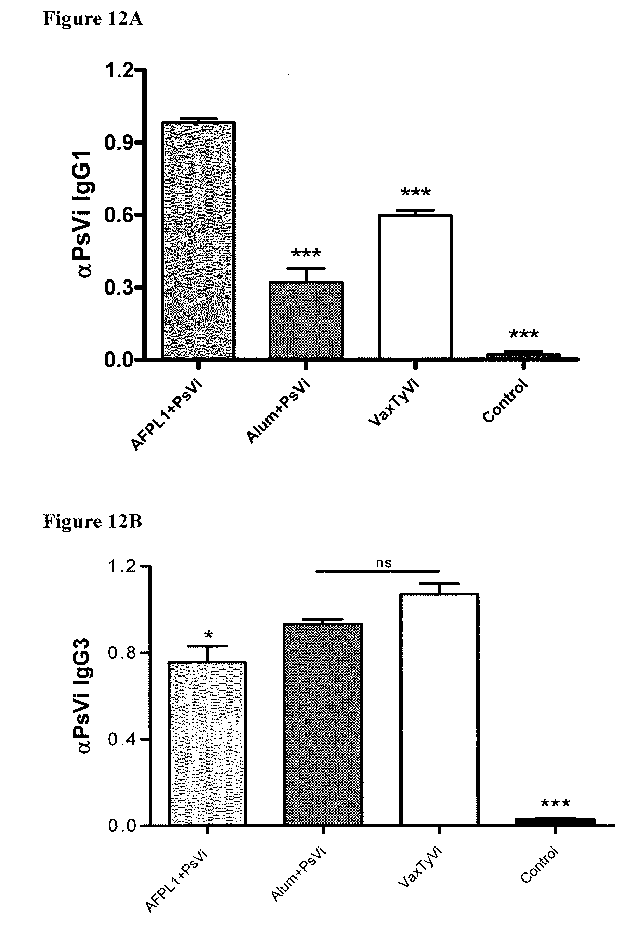

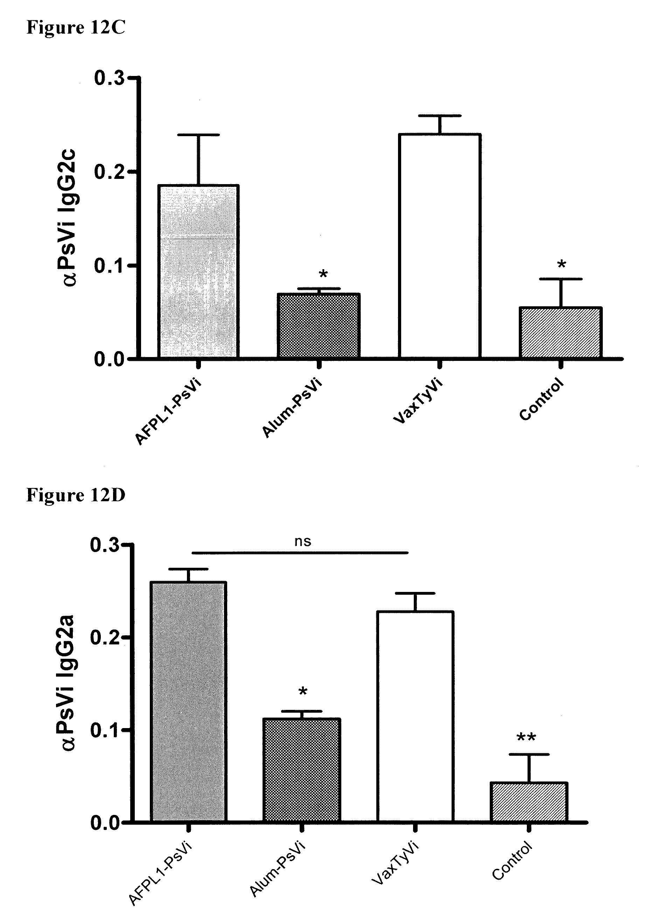

The use of AFPL1 by parenteral route induced IgG3 values lower than those obtained in the remaining groups and responses in other subclases (Fig. 12A, 12B,12C and 12D).

### Conclusions

Adjuvant AFPL1 by parenteral route permits IgG3 induction, typical subclass of the response of non conjugated TI-2 antigens.

### Example 14. Determination of the affinity index and accumulated reduction percentage with different concentrations of Thiocyanate for intramuscular formulation of AFPL1 and PsVi

### Immunization protocol and sample collection. See example 12.

### Detection of the affinity index of anti PsVi antibodies by avidity ELISA test

### Reagents and solutions:

- Polysaccharide Vi was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Solution with chaotropic agent with Potassium Thiocyanate at different molar concentrations: KSCN 0.1M, 0.25M, 0.5M and 1M
- Anti Mouse IgG conjugated to peroxidase (SIGMA, St. Louis, MO, EUA);
- Substrate buffer solution (STS): Na₂HPO₄ 52 mM, citric acid 25 mM (pH 5,6), and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of Polysaccharide at a concentration of 10 µg/mL for PsVi while CBS is added and incubate overnight at 4°C in wet chamber ;
- Wash three times with PBSS;
- Add 200 µL/well of SB and incubate 1 h at room temperature in wet chamber.
- Wash three times with WS;
- Dilute sera 1:25 in SDS and apply 100 µL/well in duplicate of each diluted samples and incubate 2 h at 37°C;
- Wash three times with WS;
- Add 100 µL/well in duplicate the different concentrations of chaotropic agent (0.1M, 0.25M, 0.5M and 1M) and a part of the plate is left with the samples that only have PBSS;
- Incubate at room temperature for 15 minutes;
- Wash three times with WS;
- Add 100 µL/well of anti mouse IgG peroxidase conjugate dilute 1:2500 in SDS
- Incubate 2 h a 37°C in wet chamber ;
- Wash five times with WS;
- Add 100 µL/well of solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- Absorbance measurement (DO) is carried out at a wavelength of 492 nm in a microplate reader (Titertek, Multiskan Plus).

### Expression and Calculation of results

Results of affinity maturing of antibodies for a Potassium Thiocyanate concentration were expressed as avidity index (%) that is calculated as the titer of the serum treated with Thiocyanate into the titer of serum not treated with Thiocyanate (PBS+Tween) by 100 and the reduction percentage of absorbance was expressed for each sample and with each Thiocyanate concentration

### Results

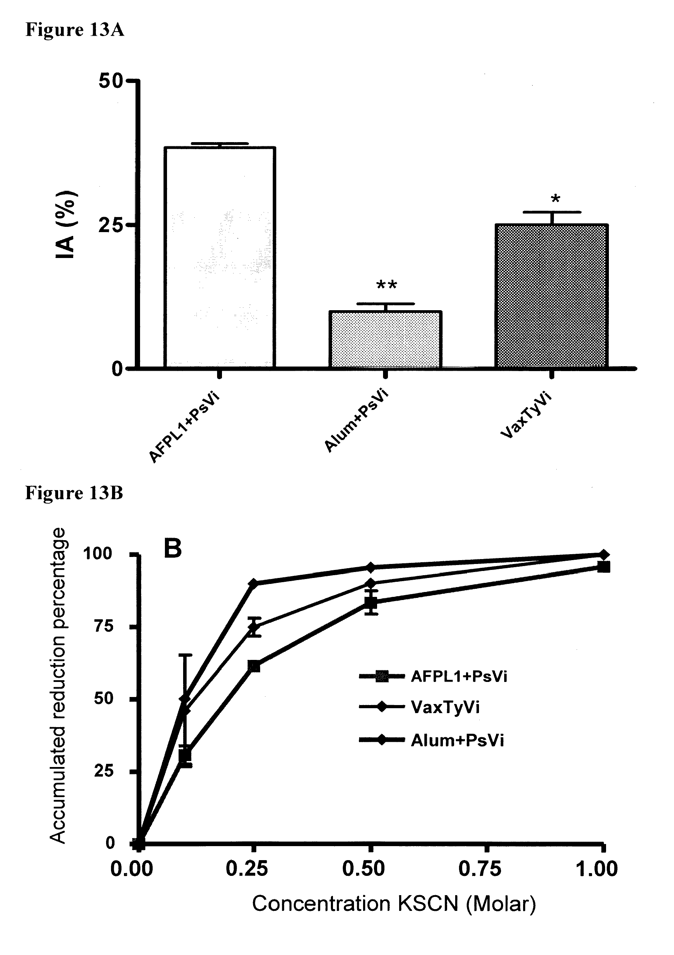

With the formulation of adjuvant and polysaccharide by intramuscular route, an affinity index superior to that of polysaccharide vaccine or when aluminum hydroxide is used as adjuvant is induced. (Fig. 13A and 13B).

### Conclusion

The greater affinity of the serum samples of the group immunized with adjuvant AFPL1 by intramuscular route Finlay and PsVi suggests a stronger formation of the antigen-antibody complex

### Example 15. Immunogenicity of formulation of outer membrane vesicles (Proteoliposome, PL) derived from N. meningitidis serogroups A and W135 coadministered with polysaccharide A from N. meningitidis serogroup A (PsA)

**Immunization protocol.** To evaluate immunogenicity of formulations, Balb/c mice were immunized with two subcutaneously doses (s.c) of 100 µL, with 2.5 µg of each PL derived from *N. meningitidis* serogroups A and W135 (PLA and PLW135, respectively) coadministered with 5 µg of PsA. Control groups were only PsA or with adjuvant Aluminum phosphate at a PsA final dose of 5 µg in 100 µL, PLA and PLW135 without capsular polysaccharide (2.5 µg each) and PsA conjugated vaccine (MenAfriVac) at a PsA dose of 0.2 µg of in 100 µL by s.c. route

**Obtainment method and sample processing.** Blood was extracted 15 days after the last dose. For serum obtainment, blood was extracted by puncture of retro-orbital plexus using heparinized capillaries. Obtained blood samples were incubated for 1 h at 37°C, then, they were centrifuged at 12500 rpm for 10 min to extract serum. Serum was stored at -20°C until use.

### Detection of anti PsA IgG and IgM by ELISA

### Reagents and solutions

- Polysaccharide A was manufactured by the Production Plant from Finlay Institute, respectively, under conditions of good manufacturing practices
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG conjugated to peroxidase (SIGMA, St. Louis, MO, EUA);
- Anti Mouse IgM conjugated to peroxidase (SIGMA, St. Louis, MO, EUA);
- Substrate buffer solution (STS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of Polysaccharide at a concentration of 10 µg/mL for PsA while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each of the diluted samples and incubate 2 h at 37°C;
- Wash three times with WS;
- Add 100 µL/well of anti Mouse IgG peroxidase conjugate diluted 1:2500 or anti Mouse IgM peroxidase conjugate diluted 1:2500 in SDS;
- Incubate 1 h at 37°C in wet chamber ;
- Wash five times with WS;
- Add 100 µL/well of solution of hydrogen peroxide 0,01% (v/v) and 6 mg/mL of ortho-phenylendiamine (OPD) en STS;
- Incubate in the dark for 30 min;
- The reaction is stopped by adding 50 µL of stopping solution and
- Absorbance measurement (DO) is carried out at a wavelength of 492 nm in a microplate reader (Titertek, Multiskan Plus).

### Expression and Calculation of results

Results of anti PsA IgG were expressed as OD. In this assay, samples are considered positive above the mean values plus 3 standard deviations of control samples (groups without immunization).

### Results

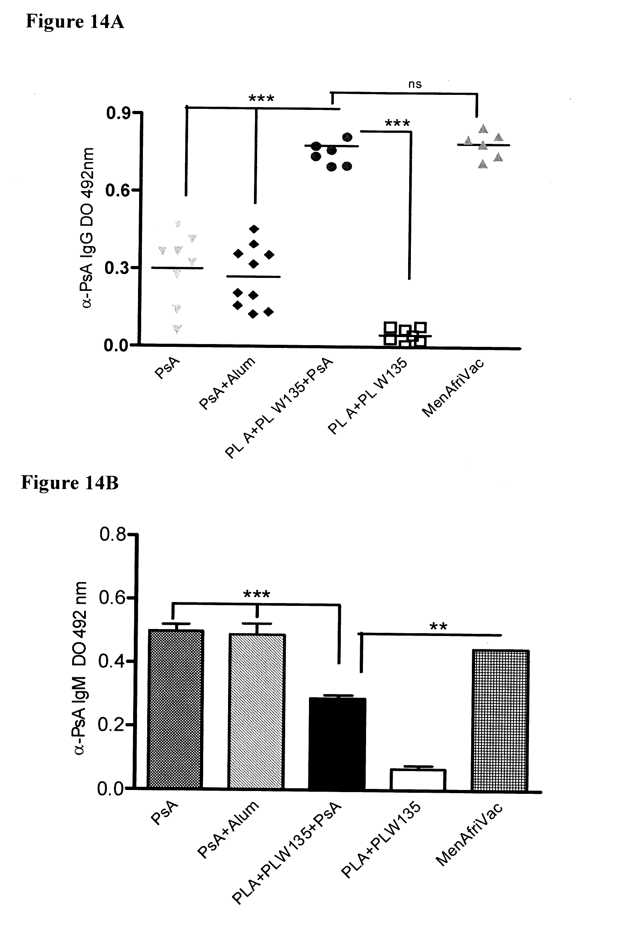

A systemic anti PsA IgG response is induced which is significantly superior to the one reached by plain polysaccharide and similar to that induced by conjugated vaccine MenAfriVac of the mentioned polysaccharide, that at the same time has aluminum phosphate as adjuvant (Fig. 14A). The primary response characterized by IgM induction was superior in group immunized with plain polysaccharide (Fig. 14 B)

### Conclusions

The predominant immune response produced by polysaccharide vaccines is IgM type, where there is a very few specific IgG response, being this response only different when the polysaccharide is conjugated with a carrier protein. The use of formulations containing potente adjuvants like PL combined with plain PsA without covalent conjugation permits to stimulate a high systemic antigen sspecific IgG response against PsA and induce lower levers of IgM type response , similar to the response obtained with commercial conjugated vaccine of this polysaccharide.

### Example 16. Determination of anti PsA bactericidal antibody titers in serum of the formulation of outer membrane vesicles (Proteoliposome, PL) derived from N. meningitidis serogroup A and W135 coadministered with polysaccharide A from N. meningitidis (PsA)

### Immunization protocol and sample collection. See example 15

### Reagents and solutions

- Complement of young rabbit (Pel Freez lot 08432);
- Hank's Balanced Saline Solution (HBSS) (Sigma lot RNBB 6657);
- Bovine Serum Albumin 10% (BSA) (Sigma lot 119K8412);

### Methodology

- In sterile 96 well plates for tissue culture with U bottom (Becton Dickinson, EUA), Add 20 µL/well of HBSS with BSA 0.1%, from column 1 up to 11 and 10 µL/well in column 12.
- Add 20 µL of serum to evaluate in column 1. Do double serial dilutions up to column 9.
- Add 10 µL/well of young rabbit complement from column 1 to 10.
- Add 10 µL/well of young rabbit complement heat at 56°C for 30 minutes in columns 11 and 12.
- Add 10 µL/well of cellular suspension containing as the blank strain of the reaction in the bactericidal assay Mk499/03 (serogroup A) and incubate 1 h at 37°C.
- Extract 10 µL/well and seed in 5% sheep blood agar plates by sloping plate method
- Titer determination is carried out by colony counting using the program Sorcerer associated to an automated colony counter. (Perceptive Instrument).

### Expression and calculation of results.

Titers were expressed as the inverse of the greater serum dilution that cause lysis at 50% or more of cells in respect to the suspension control.

### Results

Serum bactericidal activity in mice immunized by s.c with two doses (0 and 21 days), of plain PsA adjuvated with PLA+PsW or conjugated were evaluated at 0, 21 and 35 days. A similar bactericidal activity was observed between the conjugated and the Pls adjuvated groups. Besides, bactericidal activity was induced in the group that, without PsA, contained PLA (Table. 1).

### Conclusions

Formulations of adjuvated Ps with potent adjuvants like PL induce serum bactericidal activities similar to the conjugated vaccine MenAfriVac.

### Example 16. Determination of pattern of anti PsA IgG1, IgG3, and IgG2a subclasses for intramuscular formulation of outer membrane vesicles from A and W135 coadministered with PsA

### Immunization protocol and sample collection. See Example 15

### Detection of anti Polysaccharide IgG1, IgG3 and IgG2a sSubclasses by ELISA test

### Reagents and solutions:

- PsA was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG3 and IgG2c conjugated to peroxidase (Southern Biotech, Birmingham, EUA);
- Anti mouse biotinylated IgG1 and IgG2a (SIGMA, St. Louis, MO, EUA)
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of polysaccharide at a concentration of 10 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:50 in SDS and apply 100 µL/well in duplicate of each of the diluted samples and incubate 2 h at 37°C;
- Wash three times with WS;
- Add 100 µL/well of the anti mouse IgG1 and IgG2a biotinylated conjugate diluted 1:5000 en SDS;
- Add 100 µL/well of anti mouse IgG3 peroxidase diluted 1:6000 in SDS
- Incubate 2 h at 37°C in wet chamber;
- Wash five times with WS;
- For IgG1 e IgG2a that are biotinylated, wash plates three and add 100 µL/well of the second conjugate (Streptavidine-peroxidase) 1:2500 in SDS and incubate for 30 min at 37°C.
- Add 100 µL/well of a solution of hydrogen peroxide 0,01% (v/v) and 6 mg/mL of ortho-phenylendiamine (OPD) in SBS;
- Incubate in the dark for 30 min;
- The reaction is stopped by adding50 µL stopping solution and
- Absorbance measurement (DO) is carried out at a wavelength of 492 nm in a microplate reader (Titertek, Multiskan Plus).

### Expression and calculation of results

Results of anti PsVi IgG1, IgG3, IgG2a subclasses were expressed as OD. In this assay, samples are considered positive above the mean values plus 3 standard deviations of control samples (groups without immunization).

### Results

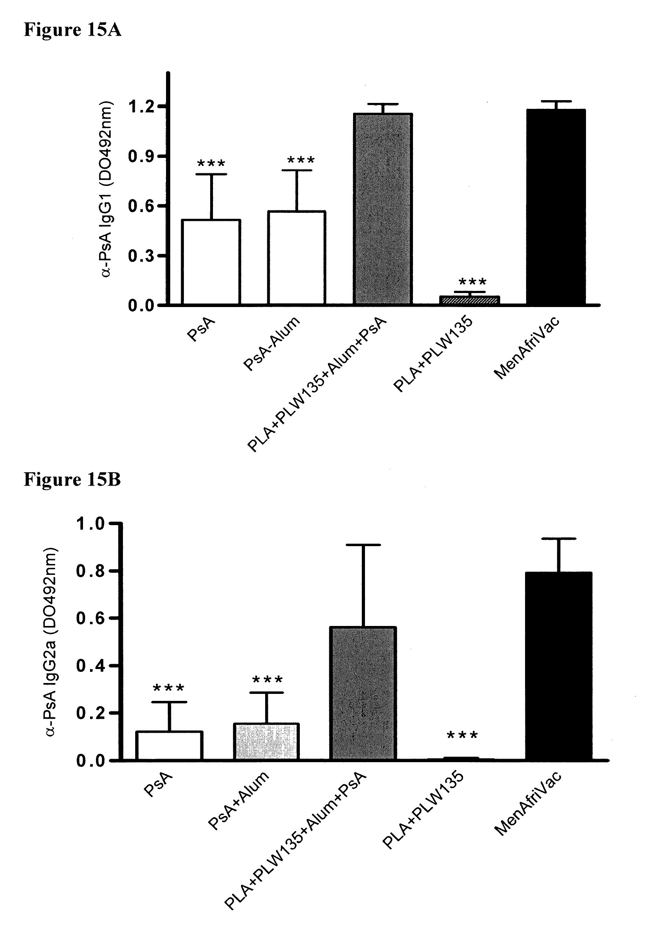

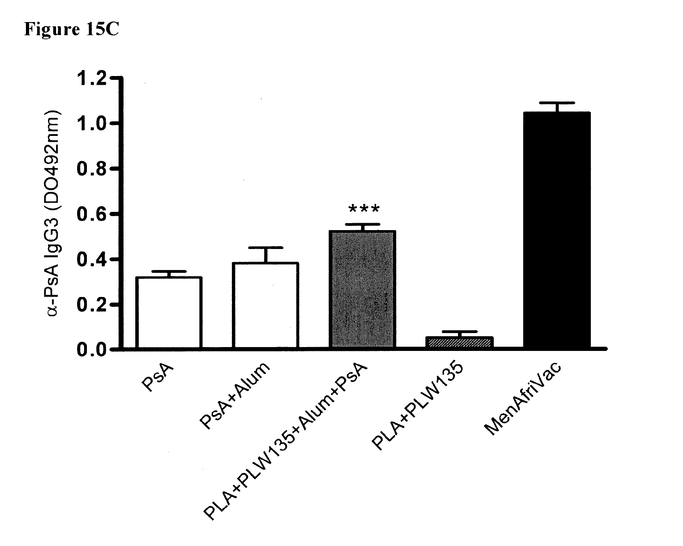

The use of PL formulated with PsA by subcutaneous route induced high values of IgG1 (Fig. 15A) and anti-PsA IgG2a (Fig. 15B) which are similar to MenAfric though with less IgG3 responses (Fig. 15C).

### Conclusions

The use of PsA combined with PLW135 as adjuvants of PsA by subcutaneous route permitted the induction of a mixed pattern of specific polysaccharide subclasses; this type of response of subclasses is typical of TD antigens, since TI-2 antigens induce a restricted response of subclass being the dominant subclass. The induction of an anti PsA IgG2a response confirms the transition from TI-2 to TD and a polatization towards pattern Th1 as occurs with the conjugation of Ps.

### Example 17. Determination of the affinity index for the formulation of outer membrane vesicles (Proteoliposome, PL) from A and W135 and PsA

### Immunization protocol and sample collection. See Example 15

### Detection of affinity of anti PsA antibodies by avidity ELISA test

### Reagents and solutions

- PsA was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Mouse IgG conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of polysaccharide at a concentration of 10 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:25 in SDS and apply 100 µL/well in duplicate of each diluted samples and incubate 2 h at 37°C;
- Wash three times with WS;
- Add 100 µL/well in duplicate the concentration of chaotropic agent (0.25M) and a part of the plate is left with the samples that only have PBSS;
- Incubate at room temperature for 15 minutes;
- Wash three times with WS;
- Add 100 µL/well of the anti mouse IgG peroxidase conjugate diluted 1:2500 in SDS
- Incubate 2 h at 37°C in wet chamber;
- Wash five times with WS;
- Add 100 µL/well of solution of hydrogen peroxide 0,01% (v/v) and 6 mg/mL of ortho-phenylendiamine (OPD) in STS;
- Incubate in the dark for 30 min;
- The reaction is stopped by adding50 µL of stopping solution and
- Absorbance measurement (OD) is carried out at a wavelength of 492 nm in a microplate reader (Titertek, Multiskan Plus).

### Expression and calculation of results

Results of maturing of affinity of antibodies for a concentration of Potassium Thiocyanate were expressed as affinity index (%) that is calculated as the titer of the serum treated with Thiocyanate into the titer of serum not treated with Thiocyanate (PBS+Tween) by 100.

### Results

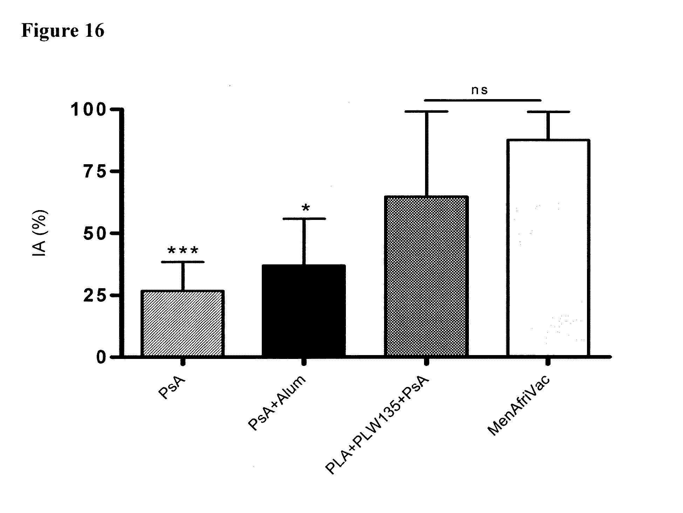

An avidity index very superior to that of plain polysaccharide was induced in PLA and PLW135 and PsA in the formulation by subcutaneous route with adjuvant Aluminium phosphate and it is similar to the affinity index induced by the conjugated commercial vaccine. (Fig. 16).

### Conclusion

The highest affinity of sera samples of the group immunized with PLA+PLW135 and PsA suggests a stronger formation of the antigen-antibody complex, maybe due to the coestimulator signals of the adjuvant , which favor the formation of germinal centers, leading to proliferation and selection of a highest number of cells B of high affinity to the epitopes of the antigen, characteristic of TD antigens and conjugated vaccines.

### Example 18. Induction of anti PsC IgG and IgM and serum bactericidal activity against N. meningitidis serogroups B and C in infants vaccinated for the first time with VA-MENGOC-BC^{®} and who currently are under one year old.

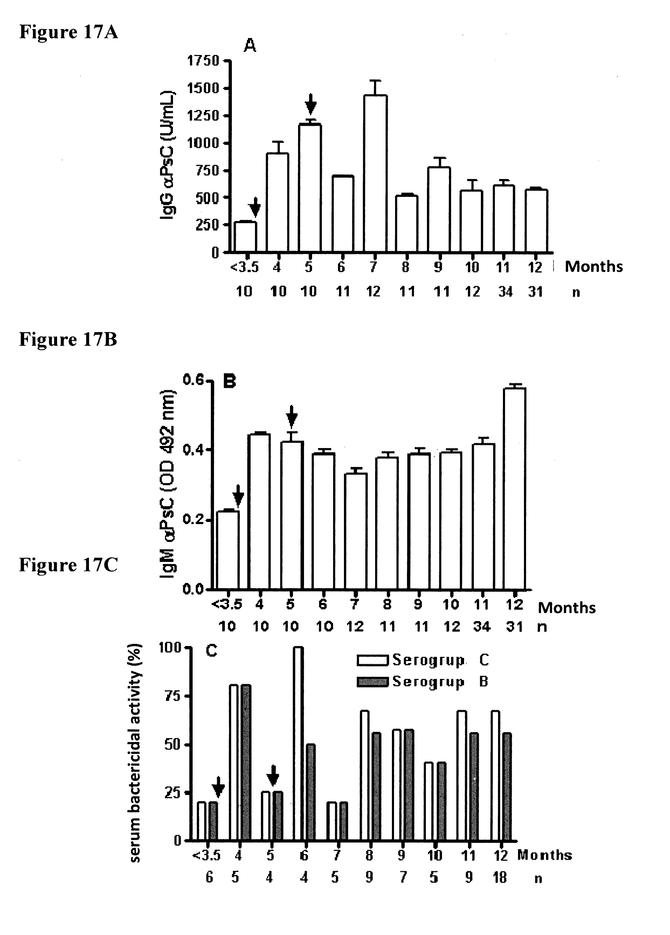

**Immunization protocol.** To evaluate immunogenicity of A-MENGOC-BC^{®}, a total of 151 infants were immunized at 3.5 months (first dose) and 5 months (second dose) with VA-MENGOC-BC^{®} containing 50 µg of PsC and 50 µg of AFPL1 adsorbed unto aluminum oxide per dose. Subsequently, the serum responses of IgG and IgM in groups at different months were evaluated. (n in Fig. 17).

**Obtainment method and sample processing.** Blood sample was carried out by venous puncture at Hospital Juan Manuel Marquez. Obtained blood samples were incubated 1 h at 37°C, then they were centrifuged at 12500 rpm for 10 min to extract the serum. Sera was stored at -20°C until use.

### Detection of anti PsC IgG and IgM by ELISA

### Reagents and solutions

- PsC was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti human IgG and IgM conjugated to peroxidase (SIGMA, St. Louis, MO, EUA);
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and Incubate 1 hour at room temperature in wet chamber;
- In 96- wells plates of high link capacity (Costar, EUA), add 100 µL/well of polysaccharide at a concentration of 2.5 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- dilute sera 1:100 in SDS and apply 100 µL/well in duplicate of each diluted sample and incubate 2 h at 37°C;
- Wash three times with WS;
- Add 100 µL/well of anti mouse IgG peroxidase conjugate diluted 1:2500 in SDS;
- Incubate 1 h a 37°C in wet chamber;
- Wash five times with WS;
- Add 100 µL/well of a solution hydrogen peroxide 0,01% (v/v) and 6 mg/mL of ortho-phenylendiamine (OPD) in SBS;
- Incubate in the dark for 30 min;
- The reaction is stopped by adding50 µL of stopping solution and
- La absorbance measurement (DO) is carried out at a wavelength of 492 nm in a microplate reader (Titertek, Multiskan Plus).

### Detection of serum bactericidal activity

Strain *N. meningitidis* serogroup C (C11) and B Cu 385 kept in skim milk (OXOID, Basingstoke, Hampshire, United Kingdom) at -70°C in multiple aliquots was used as antigen in this assay. The day before the conduction of the assay , a frozen aliquot was taken and was seed by streaking technique to obtain isolated colonies in Agar Mueller-Hinton (OXOID, Basingstoke, Hampshire, United Kingdom) plates supplemented with SBF 10% (SIGMA, St. Louis, MO, EUA) and the inhibitor vancomicin-colimicin-nistatin (VCN) (bioMerieux SA, Marcy I'Etoile, Francia). Plates were incubated overnight at 37°C in 5% of CO₂ atmosphere. One isolated colony was transferred at a plate of the same medium, previously tempered and incubated for 4 h at 37°C in 5% of CO₂. Cells were collected with physiological saline solution pH 7,2-7,4 and the suspension was adjusted spectrophotometrically at an OD in a range from from 0,5-0,55, read at 600 nm.

### Procedure

All steps of EBSc were carried out in a Biological Safety Cabinet. Sera samples were diluted in microtitering plates (Costar Laboratories, Cambridge, Mass, EUA) from 1:2 to 1:4096 in SSHB with BSA 0,1% (SIGMA, St. Louis, MO, EUA) (pH 7,2). A total of 12,5 µL/well of the cellular suspension were added, approximatedly 100 colony forming units (CFU), in all microplate wells. Subsequently, 12,5 µL of the complement were added. Plates were incubated at 37°C in atmosphere without CO₂ for 30 min. Then, 150 µL/ well of Mueller-Hinton Broth (OXOID, Basingstoke, Hampshire, United Kingdom) with glucose 2% (BDH Laboratory Supplies Poole, United Kingdom), pH indicator Purple Bromocresol 0,002% (Fluka Chemika, Suiza) and VCN were added. The Ph indicator was previously prepared at 1,6% en ethanol. The inhibitor VCN was used to ensure the absence of contaminations that may have affected the result of the assay. Plates were incubated 20 h at 37°C in 5% of CO₂ atmosphere.

### Expression and calculation of results

Results of anti PsC IgG were expressed in U/mL using an anti PsC pattern curve. Samples were considered positive above 367 U/mL. Results of anti PsC IgM were expressed as OD. In this assay, samples are considered positive above the mean values plus 3 standard deviations of control samples. The bactericidal titer was determined as the reciprocal of the maximum dilution of the serum that caused >90% of death of strain C11 or Cu 385 and that detected no color change of the pH indicator. Titration was carried out at first sight and OD was read at 405 nm in a microplate reader (Labsystems Multiskane^{®} Multisoft) to confirm the results. The results are expressed in percentages of responders.

### Results

The response anti PsC IgG was increased since the first dose and was kept positive in all evaluated groups from 4 and 12 months old (Fig. 17A). Anti PsC IgM was also kept positive in all evaluated groups (Fig. 17B). The bactericidal response was similar for serogroups B and C except at 4 months in which anti C response was predominant (Fig. 17C).

### Conclusions

IgG to IgM and anti PsC bactericidal responses were induced in first vaccination with non conjugated PsC adjuvated with AFPL1 absorbed unto aluminum oxide in infants. They lasted one year.

### Example 19. Persistence of anti PsC IgG and IgM responses in infants first vaccinated with VA-MENGOC-BC^{®} up to at least 15 years

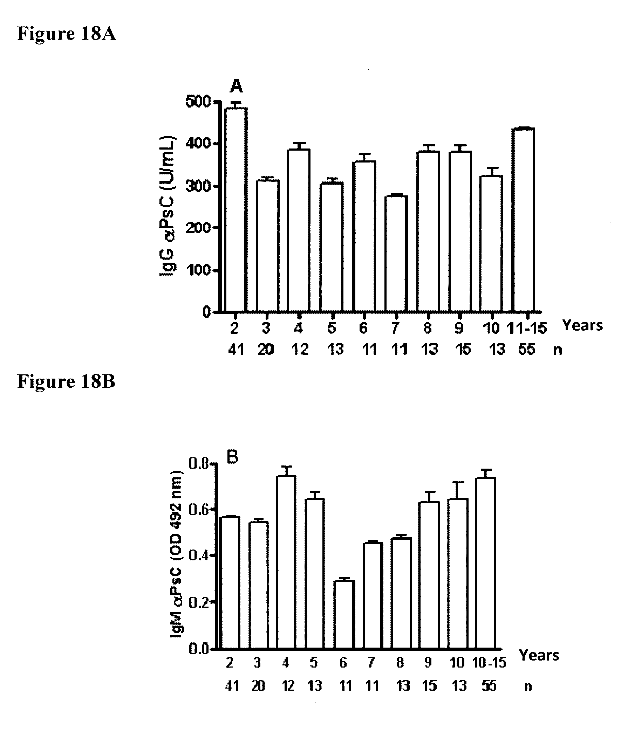

**Immunization protocol.** To evaluate the persistence of the response induced by VA-MENGOC-BC^{®}, a total of 191 infants were immunized at 3.5 months (first dose) and 5 months (second dose) with VA-MENGOC-BC^{®} containing 50 µg of PsC and 50 µg of AFPL1 adsorbed unto aluminum oxide per dose. Subsequently, the serum responses of IgG and IgM in groups at different months were evaluated. (n in Fig. 18).

Anti PsC IgG and IgM detection by ELISA was carried out as described in Example 18

### Results

Anti PsC IgG response is kept up to 15 years old though in low titers (Fig. 18A). However, there is anti PsC IgM response in all groups except in that of 6 years old (Fig. 18B).

### Conclusions

The anti PsC IgG to IgM response induced by first vaccination with a non conjugated PsC adjuvated with AFPL1 absorbed unto aluminum oxide in infants was persistant up to 15 years old, though with low titers.

### Example 20. Anti PsC IgG Subclasses and its duration in infants first vaccinated with VA-MENGOC-BC^{®} in comparison with a plain PsC vaccine.

**Immunization protocol and obtainment and processing of samples.** See description in Examples 18 and 19. Fifteen young adults vaccinated with a dose of plain A+C vaccine were additionally included.

### Detection of anti PsC IgG1, IgG2, IgG3 and IgG4 subclasses by ELISA test

### Reagents and solutions

- PsC was manufactured by the Production Plant from Finlay Institute, respectively, under conditions of good manufacturing practices;
- Cover buffer solution (CBS): Na₂CO₃ 11 mM, NaHCO₃ 35 mM (pH 9,6)
- Phosphate Buffered Saline Solution (PBSS) 0.15 M (pH 7.2);
- Block solution (BS): PBSS, 1% BSA (SIGMA, St. Louis, MO, EUA);
- Washing Solution (WS): PBSS, 0.05% Tween-20 (v/v);
- Sample dilution solution (SDS): SL, 1% BSA (SIGMA, St. Louis, MO, EUA);
- Anti Human biotinylated IgG1, IgG2, IgG3 and IgG4 (SIGMA, St. Louis, MO, EUA)
- Substrate buffer solution (SBS): Na₂HPO₄ 52 mM, citric acid 25 mM (pH 5,6) and
- Stopping solution: H₂SO₄ 2 M.

### Methodology

- In 96- wells plates of high link capacity (Costar, EUA), add 100 µL/well of Poli-L-lisine (Sigma, USA) at a concentration of 3 µg/mL, while PBSS is added and incubate 1 hour at room temperature in wet chamber;
- In 96- wells plates of high link capacity (Costar, EUA), add 100 µL/well of polysaccharide at a concentration of 10 µg/mL while CBS is added and incubate overnight at 4°C in wet chamber;
- Wash three times with PBSS;
- Add 200 µL/well of BS and incubate 1 h at room temperature in wet chamber.
- Wash three times with WS;
- Dilute sera 1:100 in SDS and apply 100 µL/well in duplicate of each diluted samples and incubate 2 h at 37°C;
- Wash three times with WS;
- Add 100 µL/well of biotinylated anti mouse subclass diluted 1:5000 in SDS;
- Incubate 2 h at 37°C in wet chamber;
- Wash five times with WS;
- Add 100 µL/well of second conjugate (Streptavidin-peroxidase) 1:2500 in SDS and incubate for 30 min at 37°C.
- Add 100 µL/well of solution of hydrogen peroxide 0,01% (v/v) and 6 mg/mL of ortho-phenylendiamine (OPD) in SBS;
- Incubate in the dark for 30 min;
- The reaction is stopped by adding 50 µL of stopping solution and
- The absorbance measurement (OD) is carried out at a wavelength of 492 nm in a microplate reader (Titertek, Multiskan Plus).

### Expression and calculation of results

Results of anti PsC IgG1, IgG2, IgG3, IgG4 subclass were expressed as OD.

### Results

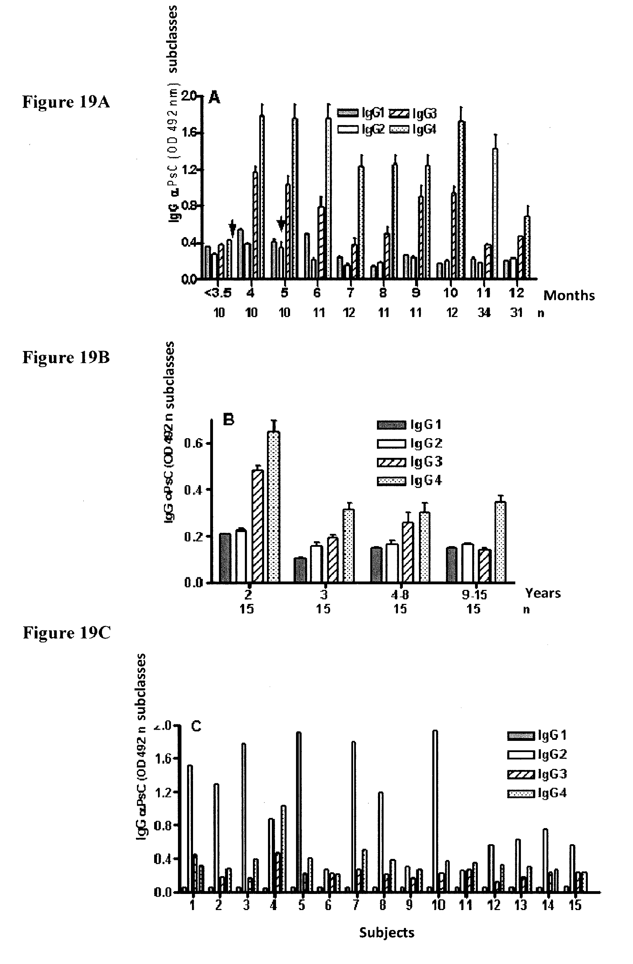

IgG4 and IgG3 were the predominant anti PsC IgG (Fig 19A) and not IgG2, which is the characteristic of plain Ps , observed in subjects vaccinated with plain vaccine (Fig. 19 C). This reponse was kept in the first year and then less up to 15 years old (Fig. 19A and 19B).

### Conclusions

Adjuvation of non conjugated plain PsC with AFPL1 adsorbed unto aluminum oxide changes the response pattern of plain Ps (IgG2) in humans and are effective in vaccination in childhood.

### Brief description of figures:

**Figure 1****. Immunogenicity of intranasal formulation of AFCo1 and AFPL1 coadministered with PsVi.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. Evaluation of anti PsVi IgG (A) and IgM (B) levels was carried out in sera extracted 15 days after the last dose, by ELISA test. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant.
**Figure 2****. Response of anti-PsVi IgG1, IgG3, IgG2a and IgG2c subclasses for intranasal formulation of AFCo1 and AFPL1 coadministered with PsVi.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of the levels anti PsVi IgG1 (A), IgG3 (B), IgG2c (C) and IgG2a (D), was carried out in sera extracted 15 days after the last dose, by ELISA test. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 3****. Determination of affinity index and affinity reduction percentage for intranasal formulation of AFPL1 or AFCo1 and PsVi.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of the avidity index of antibodies anti PsVi IgG (A) was carried out in sera extracted 15 days after the last dose by avidity ELISA test with the chaotropic agent Potassium Thiocyanate 0.25M. the evaluation of the absorbance (B) was carried out by avidity ELISA test test with different concentrations of the chaotropic agent (0.1M, 0.25M, 0.5M and 1M). The absorbance reduction percentage calculated for each concentration of Potassium Thiocyanate of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 4****. Kinetics of anti-PsVi antibodies of intranasal formulation of AFPL1 or AFCo1 coadministered with PsVi from *Salmonella* typhi (PsVi) and the secondary response after a booster with Vax-TyVi.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of anti PsVi IgG (A) levels was carried out in sera extracted 15, 30, 60, 90, 100 and 112 days after the last dose by ELISA. The comparison between the primary and the secondary anti-PsVi IgG specific response (B) was determined with sera extracted at 15 days of the first vacination scheme and at 12 days of the booster with Vax-TyVi. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant.
**Figure 5****. Determination of the affinity index and of the absorbance reduction percentage for intranasal formulation of AFPL1 or AFCo1 and PsVi after a booster dose with VaxTyVi at 100 days of the last dose** Mice C57BL/6 were immunized with 3 i.n doses (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of the avidity index of anti PsVi IgG antibodies (A) was carried out in sera extracted at 12 days after the booster with Vax-TyVi, by avidity ELISA test test with the chaotropic agent Potassium Thiocyanate 0.5M. The evaluation of the absorbance reduction percentage (B) was carried out by avidity ELISA test with different concentrations of the chaotropic agent (0.1M, 0.25M, 0.5M). The percentage of absorbance reduction calculated for each concentration of Potassium Thiocyanate of two determinations in independent experiments is shown in the figure. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 6****. Determination of pattern of anti- PsVi IgG1, IgG3, IgG2a and IgG2c subclasses for intranasal formulation of AFPL1 or AFCo1 coadministered with PsVi after a booster dose with Vax-TyVi 100 days after the last dose.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of anti PsVi IgG1 (A), IgG3 (B), IgG2c (C) and IgG2a (D) levels was carried out in sera extracted after a booster dose with Vax-TyVi, by ELISA The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 7****. Determination of effector T cells by standard ELISPOT after a booster dose with Vax-TyVi 100 days after the last dose.** Mice C57BL/6 were immunized with 3 doses i.n (25 µL) of AFCo1 or AFPL1 (50 µg) and PsVi (25µg). PsVi only by i.n route (3 dosis of 25 µg of PsVi) and the Cuban typhoid fever vaccine, Vax-TyVi (5 µg/100 µL) by intramuscular route were used as controls. The evaluation of specific secretory anti PsVi IFN-γ cells was determined in spleen sera extracted 10 days after the booster with Vax-TyVi, bye ELISA Means of two independent experiments are shown in the figure.
**Figure 8****. Determination of systemic immunogenicity of intranasal formulation of AFCo1 coadministered with Polysaccharide C from *Neisseria meningitidis* (PsC) after a nasal booster with plain PsC.** Mice Balb/c were immunized with 3 dosis i.n (25 µL) of AFCo1 (50 µg) and PsC (10 µg). PsC only by i.n route was used as control (3 doses of 10 µg). The evaluation of anti PsC IgG levels was carried out in sera extracted 21 days after the last dose by ELISA. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 9****. Determination of mucosal immunogenicity of intranasal formulation AFCo1 coadministered with PsC from *Neisseria meningitidis* after a nasal booster with plain PsC.** Mice Balb/c were immunized with 3 dosis i.n (25 µL) of AFCo1 (50 µg) and PsC (10 µg). PsC only by i.n route (3 dosis of 10 µg) was used as controls. The evaluation of anti PsC IgA levels in saliva (A) and in vaginal douching (B) was carried out in saliva and in vaginal douching extracted 7 and 28 days respectively after the last dose by ELISA. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 10****. Determination of the pattern of anti Polysaccharide IgG1, IgG2a subclasses for intranasal formulation of AFCo1 coadministered with PsC after a nasal booster with plain PsC.** Mice Balb/c were immunized with 3 dosis i.n (25 µL) of AFCo1 (50 µg) and PsC (10 µg). PsC only by i.n route (3 dosis of 10 µg) was used as control. The evaluation of anti PsC IgG1 and IgG2a levels was carried out on sera extracted 21 days after the last dose, by ELISA test. The figure shows the means and standard deviations of the mathemathic relation of values (OD) of two determinations in 2 independent experiments.
**Figure 11****. Immunogenicity of the formulation by intramuscular route of AFPL1 and PsVi.** Mice C57BL/6 were immunized with 1 dose i.m (100 µL) of AFPL1 (12 µg) and PsVi (5 µg). PsVi adsorbed unto aluminum oxide (5 µg of PsVi and 400µg of Aluminum oxide) and the Cuban typhoid fever vaccine, Vax-TyVi (5 µg/100µL) were used as control, both of them as a single intramuscular dose. The evaluation of the levels of anti PsVi IgG (A) and IgM (B) was carried out in sera extracted 15 days after the last dose, by PsC only by i.n route (3 dosis of 10 µg) was used as controls ELISA. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 12****. Response of anti-PsVi IgG1, IgG3, IgG2a and IgG2c subclasses for intramuscular formulation of AFPL1 amd PsVi.** Mice C57BL/6 were immunized with 1 dose i.m (100 µL) of AFPL1 (12 µg) and PsVi (5 µg). PsVi adsorbed unto aluminum oxide (5 µg of PsVi and 400µg of Aluminum oxide) and the Cuban typhoid fever vaccine, Vax-TyVi (5 µg/100µL) were used as control, both of them as a single intramuscular dose. The evaluation of anti PsVi IgG1 (A), IgG3 (B), IgG2c (C) and IgG2a (D) levels , was carried out in sera extracted 21 days after the last dose, by ELISA The figure shows the means and standard deviations of the mathemathic relation of values (OD) of two determinations in 2 independent experiments. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 13****. Determination of the affinity index and of the affinity reduction percentage for intramuscular formulation of AFPL1 and PsVi.** Mice C57BL/6 were immunized with 1 dose i.m (100 µL) de AFPL1 (12 µg) and PsVi (5 µg). PsVi adsorbed unto aluminum oxide (5 µg of PsVi and 400µg of Aluminum oxide) and the Cuban typhoid fever vaccine, Vax-TyVi (5 µg/100µL) were used as control, both of them as a single intramuscular dose. The evaluation of the avidity index of anti PsVi IgG antibodies (A) was carried out in sera extracted 15 days after the last dose, by avidity ELISA test with chaotropic agent Potassium Thiocyanate 0.25M. The evaluation of the absorbance reduction percentage (B) was carried out by avidity ELISA test with different concentrations of chaotropic agent (0.1M, 0.25M, 0.5M and 1M). The percentage of absorbance reduction calculated for each concentration of Potassium Thiocyanate of two determinations in independent experiments is shown in the figure. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 14****. Immunogenicity of the formulation of outer membrane vesicles (Proteoliposome, PL) from *N. meningitidis* A and W135 by subcutaneous route and Polysaccharide A (PsA) from *N. meningitidis* serogroup A.** Mice Balb/c were immunized with 2 doses (100 µL) subcutaneous (s.c) of outer membrane vesicle from *N. meningitidis* serogroup A and W135 (2.5 µg each) and PsA (5µg). PsA only and adsorbed unto Aluminum phosphate (5 µg of PsA and 400 µg of aluminum oxide Alumina) and the conjugated commercial vaccine Polysaccharide A, MenAfriVac (2 µg/100µL) were used as controls, all in 2 s.c doses. The evaluation of the levels of anti PsA IgG (A) and IgM (B) was carried out in sera extracted 15 days after the last dose, by ELISA. The figure shows the means and standard deviations of the mathemathic relation of values (OD) of two determinations in two independent experiments. Significant differences among the means of the different groups were determined by Tukey's multiple comparison test, present in Graph Pad Prism 4 software (Calif.). *P* <0.05 value was considered statistically significant.
**Figure 15****. Response of anti PsA IgG1, IgG3 and IgG2a subclasses for formulation with outer membrane vesicles (Proteoliposome, PL) from *N*. *meningitidis* A and W135 with PsA.** Mice Balb/c were immunized with 2 subcutaneous (s.c) doses (100 µL) of outer membrane vesicle from *N. meningitidis* serogroup A and W135 (2.5 µg each) and PsA (5 µg). PsA only and adsorbed unto Aluminum phosphate (5 µg of PsA and 400 µg of aluminum oxide Alumina) and the conjugated commercial vaccine Polysaccharide A, MenAfriVac (2 µg/100µL) were used as controls, all in 2 s.c doses. The evaluation of the levels of anti PsA IgG1 (A), IgG3 (B), and IgG2a (C), was carried out in sera extracted 21 days después after the last dose, by ELISA The means and standard deviations of the mathemathic relation of values (OD) of two determinations in two independent experiments are shown in the figure. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 16****. Determination of the anti-PsA affinity index for formulation of outer membrane vesicles from *N*. *meningitidis* A and W135 with PsA.** Mice Balb/c were immunized with 2 subcutaneous (s.c) doses (100 µL) of outer membrane vesicles from *N. meningitidis* serogroup A and W135 (2.5 µg each) and PsA (5 µg). PsA only and adsorbed unto Aluminum phosphate (5 µg of PsA and 400 µg of aluminum oxide Alumina) and the conjugated commercial vaccine Polysaccharide A, MenAfriVac (2 µg/100µL) were used as controls, all in 2 s.c doses. The evaluation of the avidity index of anti PsA IgG antibodies (A) was carried out in sera extracted 15 days after the last dose, by avidity ELISA test with chaotropic agent Potassium Thiocyanate 0.25M. Significant differences among the means of the different groups were determined by Tukey's multiple comparison test, present in Graph Pad Prism 4 software (Calif.). *P* <0.05 value was considered statistically significant and it was represented by *** (p<0.001); ** (p <0.01) and * (p<0.05).
**Figure 17****. Response of anti Polysaccharide C (PsC) IgG and IgM from *N. meningitidis* serogroup C and serum bactericidal activity (SBA) in infants first vaccinated with VA-MENGOC-BC^{®} who are under one year old.** Each group of n sera came from a total of 151 infants who were vaccinated at 3.5 (first dose) and 5 (second dose) months. A:, anti PsC IgG; B, anti PsC IgM and C:, serum bactericidal activity against serogroups B and C from *N. meningitidis.* Arrows represent the doses with VA-MENGOC-BC^{®}, bivalent vaccine containing PsC adsorbed unto aluminum oxide with AFPL1.
**Figure 18****. Persistency of the response of anti Polysaccharide C (PsC) IgG and IgM from *N. meningitidis* serogroup C in infants first vaccinated with VA-MENGOC-BC^{®} who are from 2 to 15 years old.** Each group of n sera came from a total of 191 infants who were vaccinated at 3.5 (first dose) and 5 (second dose) months and who currently are children (136) and schoolchildren and teenagers (55). A: anti PsC IgG, and B: anti PsC IgM.
**Figure 19****. Subclasses of induced serum IgG and its duration in infants first vaccinated with VA-MENGOC-BC^{®}.** Each group of n sera came from a total of 388 subjects first vaccinated at 3.5 (first dose) and 5 (second dose) months who currently are 151 infants, children 136, schoolchildren and teenagers 55 and young adults 46. Besides, 15 a young adults received a dose of plain A+C vaccine (Pasteur Merieux Connaught). A: anti PsC IgG subclasses in under two years old; B: anti PsC IgG subclasses from 2 to 15 years old; and C: anti PsC IgG subclasses in those immunized with plain A+C vaccine.

## Claims

1. Immunogenic adjuvated composition containing non conjugated polysaccharide antigens for parenteral, mucosal and simultaneous application.

2. Immunogenic composition according to Claim 1, containing (a) a capsular antigen from *N. meningitidis* serogroup C or Polysaccharide Vi from *Salmonella typhi,* non conjugated, and (b) AFCo1 as adjuvant for mucosal application or AFPL1 as adjuvant for parenteral application.

3. Immunogenic composition according to Claim 1, containing: (a) a non conjugated capsular antigen from *N. meningitidis* serogroup A and (b) an homologous AFPLx (from *N. meningitidis* serogroup A) absorbed unto aluminum oxide as adjuvant for parenteral applications.

4. Immunogenic compositions according to Claims 1-3, to which a conjugated polysaccharide is added.

5. Immunogenic compositions according to Claims 2-3, containing the same adjuvant (AFPLx or AFCox) applied by both parenteral and mucosal routes.

6. Immunogenic compositions according to Claims 1 -4, where polysaccharide antigens were purified from microbes, turned into oligosaccharides, obtained from recombinant tecnhiques or were synthetic.

7. Composition of claims 1 to 4, when the composition includes only meningococcus, one, two or three saccharides from (1) meningococus, (2) *Haemophylus influenzae* and *(3) Streptococo pneumonieae.*

8. The immunogenic compositions of claims 1-7, where adjuvants, AFPLx or AFCox, polarize the response of non conjugated polysaccharide antigens towards a pattern Th1 after been administered to a subject permitting int his way, that such formulations, work in newlyborn, infants and induce immune memory.
